# EUROPEAN PATENT SPECIFICATION

(11) **EP 4 376 914 B1**
(45) Date of publication and mention of the grant of the patent: **18.03.2026**
(21) Application number: 22761676.0
(22) Date of filing: 28.07.2022
(51) Int. Cl.: A61M 1/00, A61B 17/22, A61B 17/00

(54) **MECHANICALLY RESONANT PULSE RELIEF VALVE FOR ASSISTED CLEARING OF PLUGGED ASPIRATION**
MECHANISCH RESONANTES IMPULSENTLASTUNGSVENTIL ZUR UNTERSTÜTZTEN BESEITIGUNG VON VERSTOPFTER ASPIRATION
VALVE DE DÉTENTE PULSÉE À RÉSONANCE MÉCANIQUE POUR L'ÉVACUATION ASSISTÉE D'UNE ASPIRATION BOUCHÉE

(30) Priority: 28.07.2021 US 202117387949
(43) Date of publication of application: 05.06.2024
(73) Proprietor: Stryker Corporation, Portage, MI 49002-9711 (US); Stryker European Operations Limited, Carrigtwohill, Co. Cork T45 HX08 (IE)
(72) Inventor: PORTER, Stephen, Piedmont, California 94610 (US)
(74) Representative: Ipsilon Luxembourg
(86) International application number: PCT/US2022/038643
(87) International publication number: WO 2023/009703

(56) References cited:
- WO-A1-2014/151209
- WO-A1-2021/150348

## Description

### FIELD OF THE INVENTION

The present disclosure relates generally to medical devices, more particularly, to devices for aspirating objects from the anatomy, e.g., a clot from the vasculature of the patient.

### BACKGROUND OF THE INVENTION

It is often desirable to remove tissue from the body in a minimally invasive manner as possible, so as not to damage other tissues. For example, removal of tissue from within a vasculature, such as blood clots, may improve patient conditions and quality of life.

Many vascular system problems stem from insufficient blood flow through blood vessels. One cause of insufficient or irregular blood flow is a blockage within a blood vessel referred to as a blood clot or thrombus. Blood clots or thrombi may embolize and form an embolus in a patient vasculature. Thrombi can occur for many reasons, including damage to the arterial wall from atherosclerotic disease, trauma caused by surgery, or due to other causes.

When a thrombus forms, it may effectively stop the flow of blood through the zone of formation. Sometimes such thrombi are harmlessly dissolved in the blood stream. At other times, however, such thrombi may lodge in a blood vessel where they can partially or completely occlude the flow of blood. If the partially or completely occluded vessel feeds blood to sensitive tissue such as, the brain, lungs or heart, for example, serious tissue damage may result. For example, thrombosis of one of the carotid arteries can lead to stroke, because of insufficient oxygen supply to vital nerve centers in the cranium. As another example, if one of the coronary arteries is 100% thrombosed, the flow of blood is stopped in that artery, resulting in a shortage of oxygen carrying red blood cells, e.g., to supply the muscle (myocardium) of the heart wall. Oxygen deficiency reduces or prohibits muscular activity, can cause chest pain (angina pectoris), and can lead to death of myocardium, which permanently disables the heart to some extent. If the myocardial cell death is extensive, the heart will be unable to pump sufficient blood to supply the body's life sustaining needs. Indeed, a large percentage of the more than 1.2 million heart attacks in the United States are caused by blood clots (thrombi) which form within a coronary artery.

When symptoms of an occlusion are apparent, such as an occlusion resulting in a stroke, immediate action should be taken to reduce or eliminate resultant tissue damage. Indeed, clinical data indicates that clot removal may be beneficial or even necessary to improve outcomes. For example, in the peripheral vasculature, clot removal can reduce the need for an amputation by 80 percent. The ultimate goal of any modality to treat these conditions of the arterial or venous system is to remove the blockage or restore patency, quickly, safely, and cost effectively. One approach is to treat a patient with clot dissolving drugs. These drugs, however, do not immediately dissolve the clot from the patient, and are typically ineffective after a predefined window, usually at 2-3 hours after the symptoms arise from the clot. Other approaches involve thrombectomy, i.e., the removal of the clot by aspiration, mechanical retrieval, or a combination thereof. Mechanical retrieval usually involves a deployable mesh-like grid, such as a stent retriever, and is often complicated and dangerous to perform.

Aspiration thrombectomy is generally an effective and common treatment for removing a clot from a blood vessel, especially in the case of ischemic stroke. In a typical endovascular aspiration thrombectomy procedure, a catheter is introduced into the vasculature of the patient until the distal end of a catheter is just proximal to the clot, and a vacuum is applied at the proximal end of the catheter, resulting in the ingestion and subsequent removal of at least a portion of the clot into the catheter. Most aspiration systems are susceptible to tip clogging when the clot that is being aspirated is too large for the aspiration conduit at the distal end of the catheter. Current technology for endovascular thrombectomy in ischemic stroke utilizes static loading. Once tip clogging occurs, the pressure in the system precipitously drops to a level that often results in boiling or cavitation of the aspirate within the system. As a result, water vapor is introduced into the system, thereby decreasing the efficiency of the aspiration, and in turn, making it more difficult, if not impossible, to ingest the clot into the catheter.

In some cases, the clog can be disrupted or forced to squeeze through the aspiration conduit by dynamically or cyclically loading the aspiration conduit, which involves using pressure pulsing to ingest the clogged clot. One method of cyclically loading the aspiration conduit uses a cyclically activated valve or similar configuration to achieve the pressure pulsing by blocking main stream flow. Typically, this is done by hand, or via an electro-mechanical or pneumatic valve which blocks aspirate flow to the pump for a specified time interval. In some instances, pressure sensing feedback has been suggested as a means for determining when to activate the valve. One cyclical loading method, described in Simon S, Grey CP, Massenzo T, et al., "Exploring the efficacy of cyclic vs static aspiration in a cerebral thrombectomy model: an initial proof of concept study," Journal of NeuroInterventional Surgery 2014;6:677-683 and PCT Publication WO2014151209A1 disclosing a manifold according to the preamble of claim 1, employs a venting mechanism that is automatically placed in an oscillatory pulse mode in response to the application of vacuum to the aspiration conduit. However, these methods either require user intervention to cyclically load the aspiration conduit in response to the realization that the aspiration conduit it has been clogged, which would distract the user from performing the aspiration procedure at hand, or cyclically load the aspiration conduit immediately upon the application of vacuum to the aspiration conduit, and thus, decreases the efficiency of the aspiration procedure during free flow (i.e., when the aspiration conduit is not clogged).

There, thus, is an ongoing need for a technique that only cyclically loads an aspiration conduit of an aspiration catheter during no-flow or low-flow conditions.

### SUMMARY OF THE INVENTION

The present invention is directed to a manifold according to claim 1. Further developments of the invention are according to dependent claims 2-15.

Other and further aspects and features of embodiments of the disclosed inventions will become apparent from the ensuing detailed description in view of the accompanying figures.

### BRIEF DESCRIPTION OF THE DRAWINGS

The drawings illustrate the design and utility of preferred embodiments of the disclosed inventions, in which similar elements are referred to by common reference numerals. It should be noted that the figures are not drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention, which is defined only by the appended claims. In addition, an illustrated embodiment of the disclosed inventions needs not have all the aspects or advantages shown. Further, an aspect or an advantage described in conjunction with a particular embodiment of the disclosed inventions is not necessarily limited to that embodiment and can be practiced in any other embodiments even if not so illustrated.

In order to better appreciate how the above-recited and other advantages and objects of the disclosed inventions are obtained, a more particular description of the disclosed inventions briefly described above will be rendered by reference to specific embodiments thereof, which are illustrated in the accompanying drawings. Understanding that these drawings depict only typical embodiments of the invention and are not therefore to be considered limiting of its scope (the scope of the invention being limited to the scope of the appended claims), the invention will be described and explained with additional specificity and detail through the use of the accompanying drawings in which:
**Fig. 1** is a block diagram of one embodiment of an aspiration system constructed;
**Fig. 2** is a plan view of an exemplary aspiration catheter used in the aspiration system of **Fig. 1****;**
**Fig. 3** is a plan view of the distal end of the aspiration catheter of **Fig. 2** in use for aspirating an occlusion from the vasculature of a patient;
**Fig. 4** is a timing diagram illustrating the negative pressure differential between a pressurized fluid source and an aspiration flow path created in the aspiration system of **Fig. 1** over time;
**Fig. 5** is a block diagram of one example of a passive pressure oscillation assembly used in the aspiration system of **Fig. 1****;**
**Fig. 6** is a block diagram another example of a passive pressure oscillation assembly used in the aspiration system of **Fig. 1****;**
**Fig. 7** is a block diagram of still another example of a passive pressure oscillation assembly used in the aspiration system of **Fig. 1****;**
**Fig. 8A** is a plan view of one example of a passive pressure oscillation assembly used in the aspiration system of **Fig. 1****,** particularly showing the passive pressure oscillation assembly in a closed position;
**Fig. 8B** is a plan view of the passive pressure oscillation assembly of **Fig. 8A****,** particularly showing the passive pressure oscillation assembly in an open position;
**Fig. 9A** is a plan view of another example of a passive pressure oscillation assembly used in the aspiration system of **Fig.** 1, particularly showing the passive pressure oscillation assembly in a closed position;
**Fig. 9B** is a plan view of the passive pressure oscillation assembly of **Fig. 9A****,** particularly showing the passive pressure oscillation assembly in an open position;
**Fig. 10A** is a plan view of another example of a passive pressure oscillation assembly used in the aspiration system of **Fig. 1****,** particularly showing the passive pressure oscillation assembly in a first state;
**Fig. 10B** is a plan view of the passive pressure oscillation assembly of **Fig. 10A****,** particularly showing the passive pressure oscillation assembly in a second state;
**Fig. 10C** is a plan view of the passive pressure oscillation assembly of **Fig. 10A****,** particularly showing the passive pressure oscillation assembly in a third state;
**Fig. 10D** is a plan view of the passive pressure oscillation assembly of **Fig. 10A****,** particularly showing the passive pressure oscillation assembly in a fourth state;
**Fig. 11A** is a plan view of another example of a passive pressure oscillation assembly used in the aspiration system of **Fig. 1****,** particularly showing the passive pressure oscillation assembly in a closed position;
**Fig. 11B** is a plan view of the passive pressure oscillation assembly of **Fig. 11A****,** particularly showing the passive pressure oscillation assembly in an open position;
**Fig. 12** is a flow diagram illustrating one method, which is not part of the invention, of operating the aspiration system of **Fig. 1** to aspirate an occlusion from the vasculature of a patient;
**Fig. 13A** is a plan view of an embodiment of a passive pressure oscillation assembly used in the aspiration system of **Fig. 1****,** particularly showing the passive pressure oscillation assembly in a first state;
**Fig. 13B** is a plan view of the passive pressure oscillation assembly of **Fig. 13A****,** particularly showing the passive pressure oscillation assembly in a second state;
**Fig. 13C** is a plan view of the passive pressure oscillation assembly of **Fig. 13A****,** particularly showing the passive pressure oscillation assembly in a third state;
**Fig. 13D** is a plan view of the passive pressure oscillation assembly of **Fig. 13A****,** particularly showing the passive pressure oscillation assembly in a fourth state;
**Fig. 13E** is a plan view of the passive pressure oscillation assembly of **Fig. 13A****,** particularly showing the passive pressure oscillation assembly in a fifth state; and
**Fig. 14** is a timing diagram illustrating the negative pressure differential between a pressurized fluid source and an aspiration flow path created in the aspiration system of **Fig. 1****,** when employing the passive pressure oscillation assembly of **Figs. 13A-13E****,** over time.

### DETAILED DESCRIPTION OF THE ILLUSTRATED EMBODIMENTS

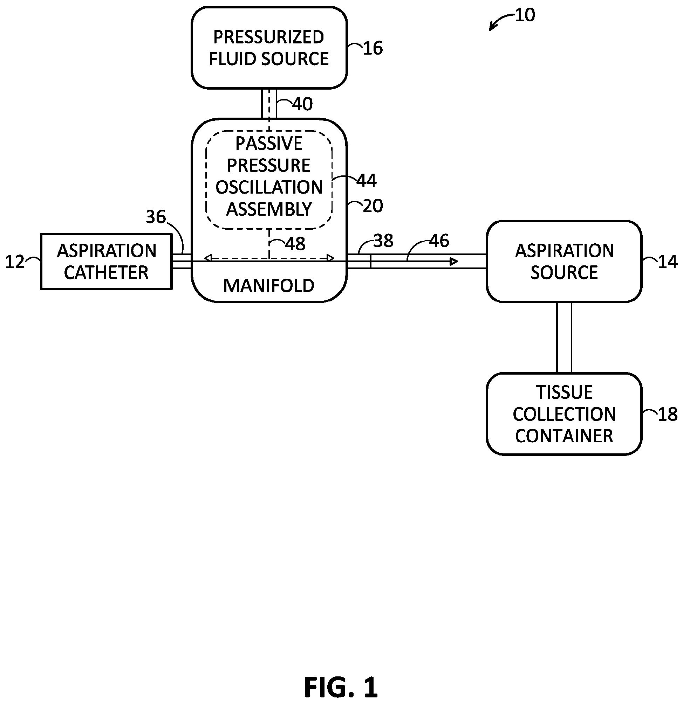

Referring to **Fig. 1****,** one embodiment of an occlusion aspiration system 10 constructed accordance with the disclosed inventions will now be described. The occlusion aspiration system 10 generally comprises an aspiration catheter 12, an aspiration source 14, a pressurized fluid source 16, a tissue collection container 18, and a manifold 20.

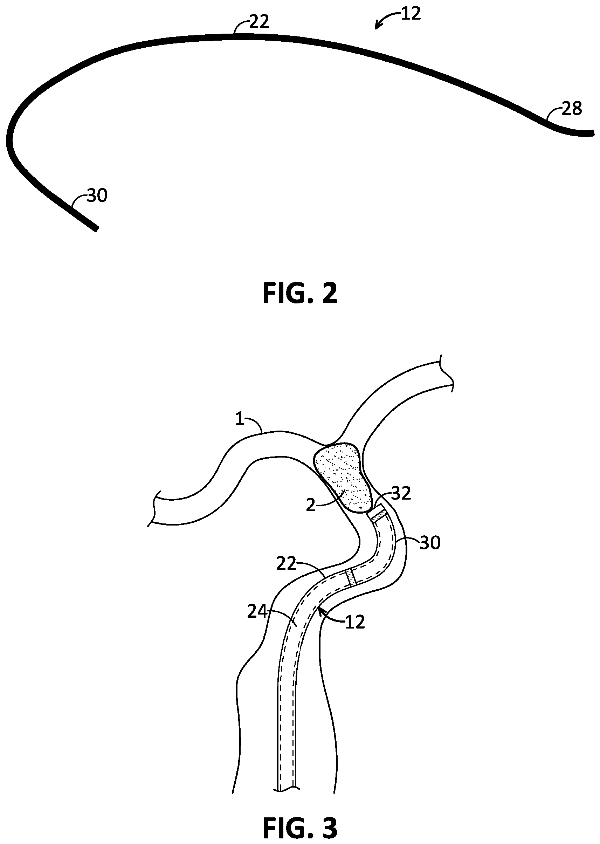

Referring further to **Figs. 2** and **3****,** the aspiration catheter 12 comprises an elongated catheter body 22, an aspiration conduit 24 (shown in phantom in **Fig. 3**) extending through the catheter body 22 between a proximal end 28 and the distal end 30 of the catheter body 22. The proximal end 28 of the aspiration catheter 12 remains outside of a patient 1 and accessible to the operator when the occlusion aspiration system 10 is in use, while the distal end 30 of the catheter body 22 is sized and dimensioned to reach an occlusion 2 (e.g., a clot) with a remote location of the vasculature 1 of the patient, as best shown in **Fig. 3****.** The aspiration catheter 12 comprises a distal inlet port 32 in communication with the aspiration conduit 24 of the aspiration catheter 12, and into which the occlusion 2 is ingested by the aspiration catheter 12.

The aspiration catheter 12 may include a plurality of regions along its length having different configurations and/or characteristics. For example, a distal portion of the catheter body 22 may have an outer diameter less than the outer diameter of a proximal portion of the catheter body 22 to reduce the profile of the distal portion of the catheter body 22 and facilitate navigation in tortuous vasculature. Furthermore, the distal portion of the catheter body 22 may be more flexible than the proximal portion of the catheter body 22. Generally, the proximal portion of the catheter body 22 may be formed from material that is stiffer than the distal portion of the catheter body 22, so that the proximal portion has sufficient pushability to advance through the vasculature 1 of the patient, while the distal portion may be formed of a more flexible material so that it may remain flexible and track more easily over a guidewire to access remote locations in tortuous regions of the vasculature 1. The catheter body 22 may be composed of suitable polymeric materials, metals and/or alloys, such as polyethylene, stainless steel or other suitable biocompatible materials or combinations thereof. In some instances, the proximal portion of the catheter body 22 may include a reinforcement layer, such a braided layer or coiled layer to enhance the pushability of the catheter body 22. The catheter body 22 may include a transition region between the proximal portion and the distal portion of the catheter body 22.

Referring back to **Fig. 1****,** the aspiration source 14 can be, e.g., conventional a pump (e.g., a rotary vane, diaphragm, peristaltic or Venturi pump) or a syringe, configured for generating a low pressure within the aspiration conduit 24 of the aspiration catheter 12. The low pressure is below the ambient air pressure, and thus, can be considered a vacuum capable of aspirating the occlusion 2 within the aspiration conduit 24 of the aspiration catheter 12. The occlusion 2 may be wholly ingested into the aspiration catheter 12 or may be broken up into pieces and ingested piece-by-piece into the aspiration catheter 12. In operation, the aspiration source 14 provides a base level of vacuum for the aspiration catheter 12. This vacuum level may be controlled and adjusted as needed by the user for aspirating tissue. Over any given time period during a tissue removal procedure, the user may set the level of vacuum to be constant or may vary the vacuum level.

The pressurized fluid source 16 may be, e.g., a reservoir containing a liquid, such as saline (e.g., a saline drip bag), or ambient air. It should be appreciated that the fluid source 16 is pressurized to the extent that the fluid has a pressure that is higher than the lowest vacuum level achieved in the aspiration conduit 24 of the aspiration catheter 12 when the aspiration source 14 is operating. Thus, even though the fluid source 16 in the illustrated example may be under low pressure (i.e., at ambient or one atmosphere absolute pressure), the fluid source 16 is pressurized relative to the pressures experienced by the aspiration conduit 24 of the aspiration catheter 12 during operation of the aspiration source 14. The tissue collection container 18 may be any suitable receptacle in fluid communication with the aspiration source 14 via an exhaust line for enabling collection and disposal of aspirated tissue in a sterile manner. Alternatively, the tissue collection container 18 may be located between the aspiration source 14 and the aspiration catheter 12.

The aspiration catheter 12, aspiration source 14, pressurized fluid source 16, and tissue collection container 18 may be conventional in nature.

In contrast, the manifold 20 is unconventional, and provides an interface between the aspiration catheter 12, aspiration source 14, and pressurized fluid source 16 in a manner that facilitates ingestion of the thrombus 2 by the aspiration catheter 12 during no-flow or low-flow conditions (e.g., if the thrombus 2 clogs the aspiration conduit 24 of the aspiration catheter 12 or otherwise there is a flow anomaly in the aspiration circuit of the system 10), while maximizing efficiency of the aspiration process during free-flow conditions (e.g., when the aspiration conduit 24 is not clogged and the aspiration circuit of the system 10 is operating as intended).

The manifold 20 comprise an aspiration inlet 36 coupled to the aspiration catheter 12, and an aspiration outlet 38 coupled to the aspiration source 14, such that an aspiration flow path 46 is formed from the aspiration catheter 12 to the aspiration source 14, and a relief inlet 40 coupled to the pressurized fluid source 16. The manifold 20 may be coupled to the aspiration catheter 12, aspiration source 14, and pressurized fluid source 16 via the use of conventional catheters (not shown) or may alternatively be integrated with the aspiration catheter 12, aspiration source 14, and pressurized fluid source 16 without the use of connectors. The manifold 20 further comprises a passive pressure oscillation assembly 44 coupled between the relief inlet 40 and the aspiration flow path 46. Significantly, the passive pressure oscillation assembly 44 is configured for dynamically loading (i.e., rapidly changing the vacuum level) the aspiration conduit 24 of the aspiration catheter 12, and in particular, cyclically loading the aspiration conduit 24 only during the no-flow or low-flow conditions. The passive pressure oscillation assembly 44 accomplishes this without user input and without the use of electronic sensors. Furthermore, the passive pressure oscillation assembly 44 may be made to be very compact, such that it can be fitted within manifold 20 will little additional bulk. The passive pressure oscillation assembly 44 may be disabled simply be blocking the relief inlet 40.

To this end, the passive pressure oscillation assembly 44 is configured for being operated between a normal mode that prevents fluid communication along a relief path 48 between the pressurized fluid source 16 and the aspiration flow path 46, such that the absolute pressure in the aspiration flow path 46 remains relatively constant and is only acted upon by the aspiration source 14, and an oscillatory mode that pulses fluid communication along the relief path 48 between the pressurized fluid source 16 and the aspiration flow path 46, such that the absolute pressure in the aspiration flow path 46 oscillates within a range of predetermined frequencies. The passive pressure oscillation assembly 44 is configured for being triggered to switch from the normal mode to the oscillatory mode in response to a clog in the aspiration conduit 24 of the aspiration catheter 12 or otherwise a flow anomaly in the aspiration conduit of the system 10, and conversely, for being triggered to switch from the oscillatory mode to the normal mode in response to removal or clearance of the clog from the aspiration conduit 24 of the aspiration catheter 12 or otherwise resolution of the flow anomaly in the aspiration circuit of the system 10. In the illustrated example, fluid communication pulsing between the pressurized fluid source 16 and the aspiration flow path 46 causes pressure pulses to propagate down the aspiration conduit 24 of the aspiration catheter 12 at the predetermined frequency. Simultaneously, fluid communication pulsing between the pressurized fluid source 16 and the aspiration flow path 46 causes fluid backflows to propagate down the aspiration conduit 24 of the aspiration catheter 12.

The passive pressure oscillation assembly 44 may be designed to pulse fluid communication along the relief path 48 between the pressurized fluid source 16 and the aspiration flow path 46 at a predetermined frequency, such that the absolute pressure in the aspiration flow path 46 oscillates at that predetermined frequency. As one example, the predetermined frequency of the pressure oscillations induced in the aspiration flow path 46 by the passive pressure oscillation assembly 44 may match the natural resonance of the fluid column within the aspiration conduit 24 of the aspiration catheter 12, such that energy transfer from the aspiration flow path 46 to the aspiration conduit 24 of the aspiration catheter 12, and thus propagation of the pressure pulses down the aspiration conduit 24 of the aspiration catheter 12, is maximized. As another example, the predetermined frequency of the pressure oscillations induced in the aspiration flow path 46 by the passive pressure oscillation assembly 44 may be selected based on the visco-elastic properties of thrombus 2 expected to be ingested by the aspiration catheter 12. That is, a clogged thrombus 2 with a softer consistency may be more susceptible to maceration, and thus subsequent ingestion, in response to relatively low-frequency, high-amplitude oscillations, whereas a clogged thrombus 2 with a harder consistency may be more susceptible to maceration, and thus subsequent ingestion, in response to relatively high-frequency, low-amplitude oscillations.

The oscillation of the passive pressure oscillation assembly 44 may optionally cause sound to emanate, and can serve as an automatic audible signal to the user that a clog in the aspiration conduit 24 of the aspiration catheter 12 has occurred. In an optional example, the passive pressure oscillation assembly 44 may be designed to pulse fluid communication between the pressurized fluid source 16 and the aspiration flow path 46 simultaneously at two or more different frequencies. For example, because determining the type of material properties of the thrombus 2 will not be known ahead of time, it may be desirable to pulse fluid communication between the pressurized fluid source 16 and the aspiration flow path 46 simultaneously at a relatively high frequency and at a relatively low frequency, such that the pressure profile in the aspiration conduit 24 of the aspiration catheter 12 is a composite of the low-frequency and high-frequency oscillations.

In the illustrated example, the passive pressure oscillation assembly 44 takes advantage of the correlation between the different flow conditions of the aspiration catheter 12 and the resulting fluid pressure levels in the aspiration flow path 46. In particular, it is expected that, in the case of a no-flow or low-flow condition where there is a clog in the aspiration conduit 24 of the aspiration catheter 12, or otherwise a flow anomaly in the aspiration circuit of the system 10, the vacuum in the aspiration flow path 46 will precipitously increase (i.e., the absolute pressure in the aspiration flow path 46 will precipitously decrease), thereby causing the negative pressure differential between the external ambient pressure and the aspiration flow path 46 to increase to a very high level (e.g., at least -55kPa), which, assuming no intervention by the passive pressure oscillation assembly 44, may even cause boiling or cavitation of the aspirate within the aspiration flow path 46 (e.g., if the such negative pressure differential below -95kPa). In contrast, it is also expected that, in the case of a free-flow condition where the aspiration catheter 12 has been unclogged, or otherwise, the flow anomaly in the aspiration circuit of the system 10 has been resolved, the vacuum in the aspiration flow path 46 will precipitously decrease (i.e., the absolute pressure in the aspiration flow path 46 will precipitously increase) to a lower level (e.g., less than -50kPa), thereby causing the negative pressure differential between the external ambient pressure and the aspiration flow path 46 to decrease to a lower level. The passive pressure oscillation assembly 44 keys off these negative pressure differentials when switching between the normal mode and the oscillatory mode.

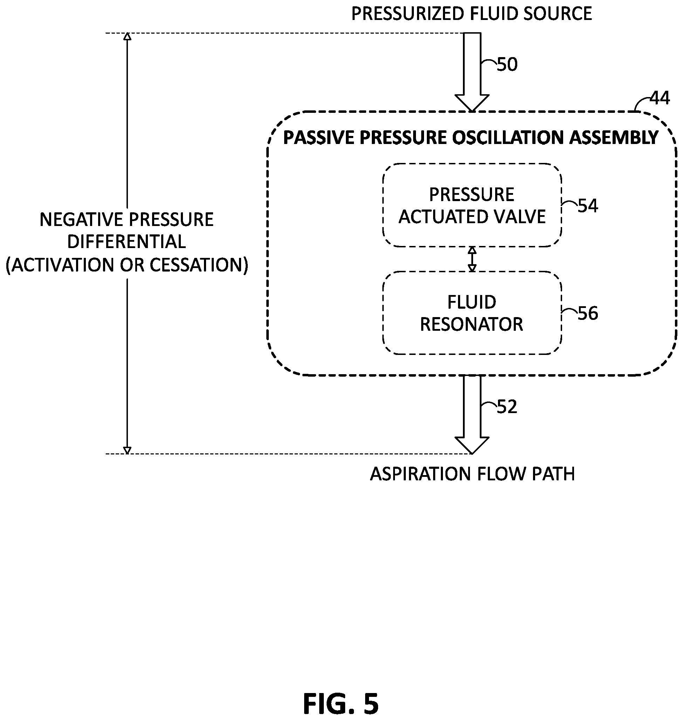

To this end, the passive pressure oscillation assembly 44 comprises an inlet port 50 (shown in **Fig. 5**) in fluid communication with the pressurized fluid source 16 and an outlet port 52 (shown in **Fig. 5**) in fluid communication with the aspiration flow path 46, such that the passive pressure oscillation assembly 44 is exposed to a negative pressure differential between pressurized fluid source 16 and the fluid in the aspiration flow path 46. The passive pressure oscillation assembly 44 is triggered to switch from the normal mode and the oscillatory mode, and conversely, from the oscillatory mode to the normal mode, based on this negative pressure differential.

In particular, the passive pressure oscillation assembly 44 is designed to be triggered to switch from the normal mode to the oscillatory mode in response to a drop in absolute pressure in the aspiration flow path 46 that creates a negative activation pressure differential between the inlet port 50 and the outlet port 52 of the passive pressure oscillation assembly 44 correlated to the negative pressure differential between the aspiration flow path 46 and the blood pressure experienced by the aspiration catheter 12 when the aspiration conduit 24 of the aspiration catheter 12 is clogged or the aspiration circuit of the system 10 otherwise experiences a flow anomaly (no-flow or low-flow condition); and conversely, the passive pressure oscillation assembly 44 is designed to be triggered to switch from the oscillatory mode to the normal mode in response to an increase in absolute pressure in the aspiration flow path 46 that creates a negative cessation pressure differential between the inlet port 50 and the outlet port 52 of the passive pressure oscillation assembly 44 correlated to the negative pressure differential between the aspiration flow path 46 and the blood pressure experienced by the aspiration catheter 12 when the aspiration conduit 24 of the aspiration catheter 12 is unclogged or the aspiration circuit of the system 10 is operating as intended (free-flow condition). Significantly, it is important that the cessation pressure differential always be negative, such that any clogged thrombus 2 does not get ejected out of the distal end 30 of the catheter body 22.

In the case where the pressurized fluid source 16 is at the external ambient pressure, the negative activation pressure differential of the passive pressure oscillation assembly 44 will essentially differ from the negative pressure differential between the aspiration flow path 46 and the blood pressure experienced by the aspiration catheter 12 by a known offset during a no-flow or low-flow condition, and likewise, the negative cessation pressure differential of the passive pressure oscillation assembly 44 will essentially differ from the negative pressure differential between the aspiration flow path 46 and the blood pressure experienced by the aspiration catheter 12 by a known offset during a free-flow condition. In this manner, the passive pressure oscillation assembly 44 may be configured to self-calibrate to a time-varying ambient external environment.

In this case, the range in which the negative activation pressure differential of the passive pressure oscillation assembly 44 is designed may have an upper limit of -55kPa, such that the passive pressure oscillation assembly 44 is quickly triggered to switch from the normal mode to the oscillatory mode, but not so low that the passive pressure oscillation assembly 44 is triggered to switch from the normal mode to the oscillatory mode during active and productive ingestion of the thrombus 2 into the distal end 30 of the aspiration catheter 12, and may have a lower limit of - 95kPa to ensure that the boiling point of fluid (i.e. blood at 37°C) in the aspiration flow path 46 is never reached, although it should be appreciated that the passive pressure oscillation assembly 44 may be designed to have a negative activation pressure differential that falls anywhere within the range of -55kPa to -95kPa. The negative cessation pressure differential of the passive pressure oscillation assembly 44 should be designed relative to the negative activation pressure differential of the passive pressure oscillation assembly 44, preferably, substantially less than the negative activation pressure differential (e.g., within the range of 10kPa-25kPa greater than the negative activation pressure differential), such that hysteresis is built into the passive pressure oscillation assembly 44. In this manner, the pressure oscillations induced in the aspiration flow path 46 by the passive pressure oscillation assembly 44 will not inadvertently trigger the passive pressure oscillation assembly 44 back into the normal mode until the aspiration catheter 12 is in a free-flow condition. It should be noted that, in low-resonant frequency scenarios, the negative activation pressure differential and the negative cessation pressure differential may be the same, in which case, the passive pressure oscillation assembly 44 will be re-triggered after each increase in the negative pressure differential in the aspiration flow path 46 to switch from the normal mode to the oscillatory mode in response to the decrease in the absolute pressure in the aspiration flow path 46 caused by the aspiration source 14.

In an optional example, the passive pressure oscillation assembly 44 may be designed with multiple negative activation pressure differentials, and correspondingly, multiple negative cessation pressure differentials. For example, the passive pressure oscillation assembly 44 may be designed to have a first negative activation pressure differential, e.g., at -50kPa, such that the passive pressure oscillation assembly 44 is triggered to switch from the normal mode to a relatively fast oscillatory mode to facilitate ingestion of the thrombus 2 into the distal end 30 of the aspiration catheter 12 prior to a clog in the aspiration conduit 24 of the aspiration catheter 12. Operation of the passive pressure oscillation assembly 44 in the relatively high oscillatory mode may cause high frequency, but low volume, pulses to propagate down the aspiration conduit 24 of the aspiration catheter 12, thereby facilitating ingestion of the thrombus 2 without overly impeding volume flow. The passive pressure oscillation assembly 44 may be further designed to have a second negative activation pressure differential, e.g., at -55kPa, such that the passive pressure oscillation assembly 44 is triggered to operate in a relatively slow oscillatory mode to facilitate clearing of a thrombus 2 that is clogged in the distal end 30 of the aspiration catheter 12. Operation of the passive pressure oscillation assembly 44 in the relatively high oscillatory mode may cause low frequency, but high volume, pulses to propagate down the aspiration conduit 24 of the aspiration catheter 12 in an attempt to dislodge the clogged thrombus 2 from the distal end 30 of the aspiration catheter 12. Thus, if the thrombus 2 is ingested without ever clogging the distal end 30 of the aspiration catheter 12, only the relatively fast oscillatory mode of the passive pressure oscillation assembly 44 will be triggered, whereas the relatively slow oscillatory mode of the passive pressure oscillation assembly 44 will only be triggered when the thrombus 2 clogs the distal end 30 of the aspiration catheter 12.

It should be appreciated that the pressurized fluid source 16 may have a pressure substantially different from the external ambient pressure experienced by the aspiration catheter 12, in which case, the negative activation pressure differential of the passive pressure oscillation assembly 44 will be substantially different from the negative pressure differential between the aspiration flow path 46 and the ambient external environment experienced by the aspiration catheter 12 during a no-flow or low-flow condition, and likewise, the negative cessation pressure differential of the passive pressure oscillation assembly 44 will be substantially different from the negative pressure differential between the aspiration flow path 46 and the ambient external environment experienced by the aspiration catheter 12 during a free-flow condition. In this latter case, this difference can be taken into account when designing the negative activation pressure differential and negative cessation pressure differential of the passive pressure oscillation assembly 44. For example, if the pressurized fluid source 16 has a pressure substantially higher than the external ambient pressure, then the passive pressure oscillation assembly 44 should be designed to a negative activation pressure differential and negative cessation pressure differential that is higher to account for the higher fluid pressure that will be experienced by the inlet port 50 of the passive pressure oscillation assembly 44.

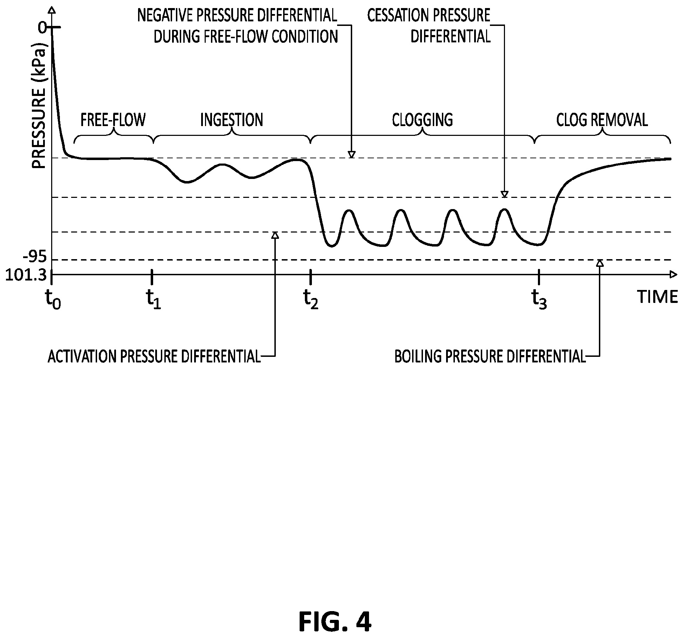

As one example, and with reference to **Fig. 4****,** the aspiration source 14 is first activated, such that the aspiration catheter 12 is in a free-flow condition between arbitrary time t₀ and t₁, and the negative pressure differential between the absolute pressure in the aspiration flow path 46 and the external ambient pressure experienced by the aspiration catheter 12 (in this case, the negative pressure differential between the inlet port 50 and the outlet port 52 of the passive pressure oscillation assembly 44) is at a free-flow negative pressure differential where the aspiration catheter 12 is only ingesting blood. During this time, the passive pressure oscillation assembly 44 remains in the normal mode. In this manner, the aspiration efficiency of the system 10 is maximized during free-flow conditions.

Between arbitrary time t₁ and arbitrary time t₂, the thrombus 2 is actively being ingested into the distal end 30 of the aspiration catheter 12, such that the negative pressure differential between the absolute pressure in the aspiration flow path 46 and the external ambient pressure experienced by the aspiration catheter 12 drops below the free-flow negative pressure differential, but not below the negative activation pressure differential of the passive pressure oscillation assembly 44, which is designed for a no-flow or low-flow condition indicative of a clogged aspiration catheter 12 or flow anomaly in the aspiration conduit of the system 10. During arbitrary time t₀ and time t₂, the passive pressure oscillation assembly 44 remains in the normal mode.

At arbitrary time t₂, however, the aspiration catheter 12 becomes clogged with the thrombus 2, resulting in a precipitous decrease in the negative pressure differential between the absolute pressure in the aspiration flow path 46 and the external ambient pressure experienced by the aspiration catheter 12 to the negative activation pressure differential, which in the illustrated case, is at -75kPa. Thus, at or just after the arbitrary time t₂, the clogged aspiration catheter 12 (no-flow or low-flow condition) triggers the passive pressure oscillation assembly 44 to switch from the normal mode to the oscillatory mode, resulting in pressure oscillations in the aspiration flow path 46 that cause pressure pulses to propagate down the aspiration conduit 24 of the aspiration catheter 12, thereby facilitating clearance of the clogged thrombus 2 at the distal end 30 of the aspiration catheter 12 at the arbitrary time t₃. Clearance of the clogged thrombus 2 at the distal end 30 of the aspiration catheter 12 results in a precipitous increase in the negative pressure differential between the absolute pressure in the aspiration flow path 46 and the external ambient pressure experienced by the aspiration catheter 12 to a level above the negative cessation pressure differential, which in the illustrated case, is at -50kPa. Thus, at or just after the arbitrary time t₃, the cleared aspiration catheter 12 (free-flow condition) triggers the passive pressure oscillation assembly 44 to switch from the oscillatory mode to the normal mode, ceasing pressure oscillations in the aspiration flow path 46, and thus ceasing pressure pulses from propagating down the aspiration conduit 24 of the aspiration catheter 12.

In the optional example where the passive pressure oscillation assembly 44 operates in multiple oscillatory modes (e.g., a high frequency oscillatory mode and a low frequency oscillatory mode), the passive pressure oscillation assembly 44 may be operated in the high frequency oscillatory mode between the arbitrary time t₁ and arbitrary time t₂, such that active ingestion of the thrombus 2 into the distal end 30 of the aspiration catheter 12 is facilitated by the high-frequency, but low volume, pressure pulses propagating down the aspiration conduit 24 of the aspiration catheter 12, and then if the thrombus 2 clogs the distal end 30 of the aspiration catheter 12, may be then operated in the low frequency oscillatory mode between the arbitrary time t₂ and the arbitrary time t₃, such that clearance of the clogged thrombus 2 from the distal end 30 of the aspiration catheter 12 is facilitated by the low-frequency, high volume, pressure pulses propagating down the aspiration conduit 24 of the aspiration catheter 12.

Referring to **Fig. 5****,** the passive pressure oscillation assembly 44 comprises a pressure actuated valve 54 and a fluid (e.g., hydraulic or pneumatic) resonator 56. The pressure actuated valve 54 is configured for opening in response to a drop in absolute pressure in the aspiration flow path 24 that creates a negative activation pressure differential between the inlet port 50 and the outlet port 52 (e.g., indicative of a clog in the aspiration conduit 24 of the aspiration catheter 12), thereby allowing the flow of fluid from the pressurized fluid source 16 through the pressure actuated valve 54; and conversely, for closing in response to an increase in absolute pressure in the aspiration flow path 24 that creates a negative cessation pressure differential between the inlet port 50 and the outlet port 52 (e.g., indicative of the removal or clearance of a clog from the aspiration conduit 24 of the aspiration catheter 12), thereby preventing the flow of fluid from the pressurized fluid source 16 through the pressure actuated valve 54. The fluid resonator 56 is configured for resonating at a predetermined frequency in response to the flow of fluid from the pressurized fluid source 16 through the pressure actuated valve 54, thereby pulsing the fluid communication between the pressurized fluid source 16 and the aspiration flow path 46 at the predetermined frequency; and conversely, configured for ceasing resonation in response to the prevention of the flow of fluid from the pressurized fluid source 16 through the pressure actuated valve 54.

In one example, the pressure actuated valve 54 and fluid resonator 56 are mechanically coupled to each other. In this example, although the mechanically coupled pressure actuated valve 54 and fluid resonator 56 must be dependently designed to satisfy both the opening and resonant frequency criteria, it results in a simpler mechanical design that can be more easily implemented into the passive pressure oscillation assembly 44. In another example, the pressure actuated valve 54 and fluid resonator 56 are mechanically decoupled from each other. In this example, the mechanically decoupled pressure actuated valve 54 and fluid resonator 56 allows the opening/closing criteria and resonant frequency criteria to be independently optimized, although resulting in a mechanical design that may be more complicated than the mechanical design of the example with the mechanically coupled pressure actuated valve 54 and fluid resonator 56.

As discussed above, the passive pressure oscillation assembly 44 may optionally be designed to have two negative activation pressure differentials, and/or two negative cessation pressure differentials, and/or pulse fluid communication between the pressurized fluid source 16 and the aspiration flow path 46 simultaneously at two different frequencies.

In an alternative example, the fluid resonator 56 pulses fluid communication between the pressurized fluid source 16 and the aspiration flow path 46 automatically in response to a clog in the aspiration catheter 12 by interrupting the aspiration flow path 46, such that the pulsing fluid communication between the pressurized fluid source 16 and the aspiration flow path 46 is directed towards the aspiration catheter 12.

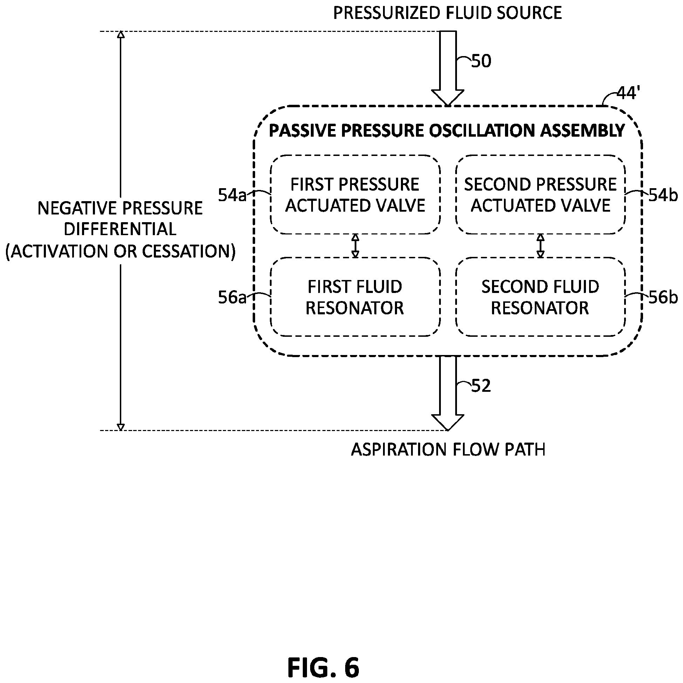

Referring to **Fig. 6****,** an alternative example of a passive pressure oscillation assembly 44' comprises two parallel sets of pressure actuated valve assemblies and fluid resonators. In particular, the passive pressure oscillation assembly 44' comprises a first pressure actuated valve 54a, a first fluid resonator 56a, a second pressure actuated valve 54b, and a second fluid resonator 56b.

The first pressure actuated valve 54a is configured for opening in response to a drop in absolute pressure in the aspiration flow path 28 that creates a first negative activation pressure differential between the inlet port 50 and the outlet port 52, thereby allowing a flow of fluid from the pressurized fluid source 16 through the first pressure actuated valve 54a; and conversely, for closing in response to an increase in absolute pressure in the aspiration flow path 28 that creates a first negative cessation pressure differential between the inlet port 50 and the outlet port 52, thereby preventing the flow of fluid from the pressurized fluid source 16 through the first pressure actuated valve 54a. The first fluid resonator 56a is configured for resonating at a first predetermined frequency in response to the flow of fluid from the pressurized fluid source 16 through the first pressure actuated valve 54a, thereby pulsing the fluid communication between the pressurized fluid source 16 and the aspiration flow path 46 at the first predetermined frequency; and conversely, configured for ceasing resonation in response to the prevention of the flow of fluid from the pressurized fluid source 16 through the first pressure actuated valve 54a.

The second pressure actuated valve 54b is configured for opening in response to a drop in absolute pressure in the aspiration flow path 28 that creates a second negative activation pressure differential between the inlet port 50 and the outlet port 52, thereby allowing a flow of fluid from the pressurized fluid source 16 through the second pressure actuated valve 54b; and conversely, for closing in response to an increase in absolute pressure in the aspiration flow path 28 that creates a second negative cessation pressure differential between the inlet port 50 and the outlet port 52, thereby preventing the flow of fluid from the pressurized fluid source 16 through the second pressure actuated valve 54b. The second fluid resonator 56b is configured for resonating at a second predetermined frequency in response to the flow of fluid from the pressurized fluid source 16 through the second pressure actuated valve 54b, thereby pulsing the fluid communication between the pressurized fluid source 16 and the aspiration flow path 46 at the second predetermined frequency; and conversely, configured for ceasing resonation in response to the prevention of the flow of fluid from the pressurized fluid source 16 through the second pressure actuated valve 54b.

The first and second negative activation pressure differentials may be the same (e.g., both indicating a clog in the aspiration conduit 24 of the aspiration catheter 12) or may be different (e.g., one indicating active thrombus ingestion into the aspiration catheter 12, and the other indicating clogging of the aspiration conduit 24 of the aspiration catheter 12). The first and second negative cessation pressure differentials may be the same (e.g., both indicating ingestion or removal of a clog in the aspiration conduit 24 of the aspiration catheter 12), although in alternative examples, the first and second negative cessation pressure differentials may be different. The first and second predetermined frequencies may be the same or may be different (e.g., one being a relatively high-frequency for disrupting a clogged thrombus 2 with a harder consistency, and the other being a relatively low-frequency for disrupting a clogged thrombus 2 with a softer consistency). The first pressure actuated valve 54a and the first fluid resonator 56a may be mechanically coupled to each other or mechanically decoupled from each other, and the second pressure actuated valve 54b and the second fluid resonator 56b may likewise be mechanically coupled to each other or mechanically decoupled from each other. Furthermore, the first pressure actuated valve 54a and the second pressure actuated valve 54b may be coupled to each other to essentially form a multi-outlet valve assembly that distributes flows to either one or the other or both of the fluid resonators 56a, 56b in response to various levels of a single sensed pressure differential.

Although the passive pressure oscillation assembly 44' is illustrated in **Fig. 6** as comprising only two parallel sets of pressure actuated valve assemblies and fluid resonators, the passive pressure oscillation assembly 44' may alternatively comprise more than two parallel sets of pressure actuated valve assemblies and fluid resonators.

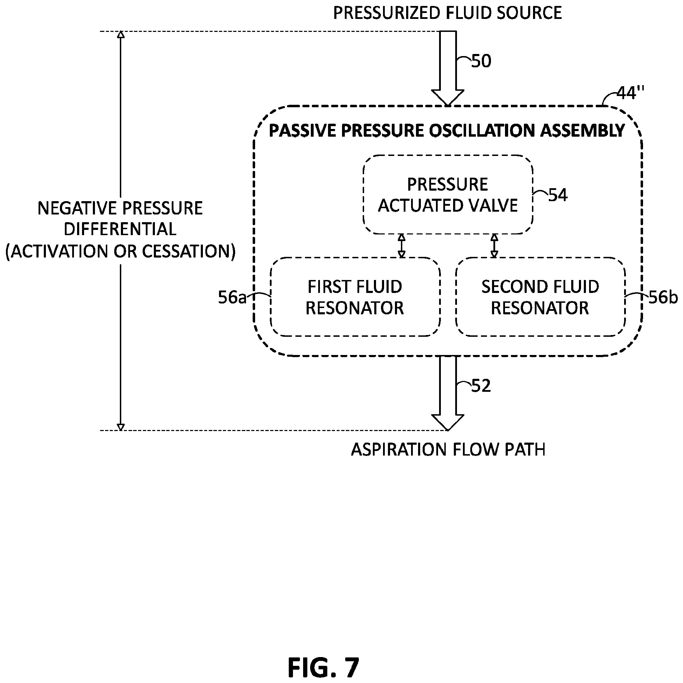

Referring to **Fig. 7****,** another alternative example of a passive pressure oscillation assembly 44" comprises a single pressure actuated valve 54, a first fluid resonator 56a, and a second fluid resonator 56b.

The pressure actuated valve 54 is configured for opening in response to a drop in absolute pressure in the aspiration flow path 28 that creates a negative activation pressure differential between the inlet port 50 and the outlet port 52, thereby allowing the flow of fluid from the pressurized fluid source 16 through the pressure actuated valve 54; and conversely, for closing in response to an increase in absolute pressure in the aspiration flow path 28 that creates a negative cessation pressure differential between the inlet port 50 and the outlet port 52 (e.g., indicative of the removal or clearance of a clog from the aspiration conduit 24 of the aspiration catheter 12), thereby preventing the flow of fluid from the pressurized fluid source 16 through the pressure actuated valve 54.

The first fluid resonator 56a is configured for resonating at a first predetermined frequency in response to the flow of fluid from the pressurized fluid source 16 through the pressure actuated valve 54, thereby pulsing the fluid communication between the pressurized fluid source 16 and the aspiration flow path 46 at the first predetermined frequency; and conversely, configured for ceasing resonation in response to the prevention of the flow of fluid from the pressurized fluid source 16 through the pressure actuated valve 54. The second fluid resonator 56b is configured for resonating at a second predetermined frequency in response to the flow of fluid from the pressurized fluid source 16 through the pressure actuated valve 54, thereby pulsing the fluid communication between the pressurized fluid source 16 and the aspiration flow path 46 at the second predetermined frequency; and conversely, configured for ceasing resonation in response to the prevention of the flow of fluid from the pressurized fluid source 16 through the pressure actuated valve 54.

The first and second predetermined frequencies may be the same or may be different (e.g., one being a relatively high-frequency for disrupting a clogged thrombus 2 with a harder consistency, and the other being a relatively low-frequency for disrupting a clogged thrombus 2 with a softer consistency). The first and second fluid resonators 56a, 56b may be mechanically decoupled from the pressure actuated valve 54.

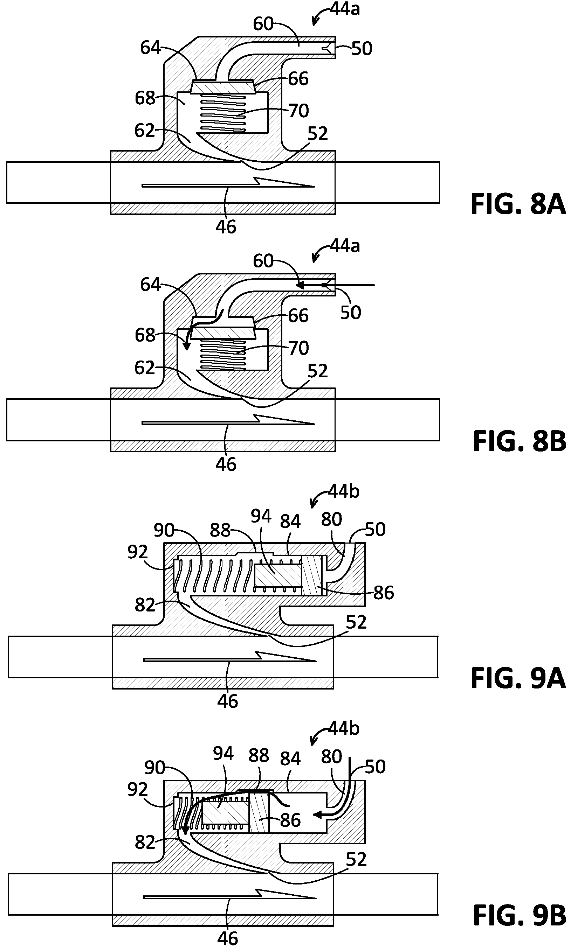

Referring now to **Figs. 8A** and **8B****,** one example of a passive pressure oscillation assembly 44a will be described. The passive pressure oscillation assembly 44a comprises an inlet channel 60 fluidly coupled to the pressurized fluid source 16 via the inlet port 50, and an outlet channel 62 fluidly coupled to the aspiration flow path 46 via the outlet port 52. The passive pressure oscillation assembly 44a further comprises a valve seal in the form of a seat 64 fluidly coupled to the inlet port 50 via the inlet channel 60, a movable valve element in the form of a valve disk 66 operably associated with the valve seat 64, and an enlarged flow cavity 68 fluidly coupled between the valve seat 64 and the aspiration flow path 46 via the outlet channel 62 and outlet port 52. The valve disk 66 is configured for being alternately displaced between a closed position to seal against the valve seat 64, and in this case, within the valve seat 64 (see **Fig. 8A**), and an open position away from the valve seat 64, and in this case outside of the valve seat 64 (see **Fig. 8B**). The passive pressure oscillation assembly 44a further comprises a restoring spring 70 affixed within the enlarged flow cavity 68 and mechanically coupled to the valve disk 66 for applying a biasing force to the valve disk 66 in a manner that maintains the valve disk 66 in the closed position within the valve seat 64 until the passive pressure oscillation assembly 44a is triggered to switch from the normal mode to the oscillatory mode, as will be described in further detail below.

The valve disk 66 and valve seat 64 have the same geometric profile (in this case, a flattened trapezoidal shape cross-section), such that the valve disk 66, when in the closed position within the valve seat 64 (see **Fig. 8A**), seals against the valve seat 64 to prevent the flow of fluid originating from the pressurized fluid source 16 (in this case, fluid that has been introduced into the inlet channel 60 via the inlet port 50) into the enlarged flow cavity 68. The enlarged flow cavity 68 has a geometric profile that is larger than the geometric profile of the valve disk 66, such that the valve disk 66, when in the open position outside of the valve seat 64 and within the enlarged flow cavity 68 (see **Fig. 8B**), allows the flow of fluid originating from pressurized fluid source 16 (in this case, fluid that has been introduced into the inlet channel 60 via the inlet port 50), into the enlarged flow cavity 68, through the outlet channel 62, and into the aspiration flow path 46 via the outlet port 52.

In response to a clog in the aspiration conduit 24 of the aspiration catheter 12, or otherwise the occurrence of an anomaly in the aspiration circuit of the system 10, a no-flow or low-flow condition occurs in the aspiration flow path 46, and as a result, the absolute pressure in the aspiration flow path 46 drops to a level that creates a negative activation pressure differential between the inlet port 50 (and thus the inlet channel 60) and the outlet port 52 (and thus the enlarged flow cavity 68), which causes the fluid in the inlet channel 60 to apply an opposing force to the valve disk 66 that overcomes the biasing force applied by the restoring spring 70 to the valve disk 66. As a result, the valve disk 66 is displaced from the closed position (see **Fig. 8A**) to the open position (see **Fig. 8B**). The negative activation pressure differential of the passive pressure oscillation assembly 44a will be based on the area of the valve disk 66 exposed to the fluid in the inlet channel 60 (the negative activation pressure differential will decrease in proportion to the exposed area of the valve disk 66) and the spring constant of the restoring spring 70 (the negative action pressure differential will increase in proportion to the spring constant of the restoring spring 70). Thus, the negative activation pressure differential of the passive pressure oscillation assembly 44a may be selected by judicially selecting the exposed area of the valve disk 66 and the spring constant of the restoring spring 70.

The passive pressure oscillation assembly 44a is designed in such a manner that, once the valve disk 66 is displaced from the closed position to the open position (i.e., the valve "cracks"), the passive pressure oscillation assembly 44a resonates (i.e., the valve disk 66 alternately switches (oscillates) between the closed position and the open position. At this point, the passive pressure oscillation assembly 44a has been triggered to switch from the normal mode to the oscillatory mode.

In particular, the biasing force applied by the restoring spring 70 to the valve disk 66, the opposing force applied by the fluid in the inlet channel 60 to the valve disk 66, and the mass of the valve disk 66 are selected, such that the valve disk 66 oscillates between the closed position and the open position at a predetermined frequency (e.g., in the range of 0.2Hz-10Hz). Preferably, the predetermined frequency is less than the natural frequency of the catheter 12, which are flexible, and as such, is susceptible to compressive shortening and lengthening upon pressure cycling. Thus, the predetermined frequency should not correspond to the natural frequency of the catheter 12, which may otherwise cause the catheter 12 to act in a spring-like axial compression/de-compression mode.

That is, when the valve disk 66 initially reaches the fully open position, the opposing force applied to the valve disk 66 by the fluid flowing from the inlet channel 60, through the valve seat 64, and into the enlarged flow cavity 68, drops to a level, such that the biasing force applied by the restoring spring 70 overcomes the opposing fluid force applied to the valve disk 66 and the momentum of the valve disk 66. As a result, the valve disk 66 is displaced from the open position back into the closed position within the valve seat 64 (see **Fig. 8A**). At this point, the momentary flow of fluid from the pressurized fluid source 16 into the aspiration flow path 46 (via the inlet port 50, inlet channel 60, valve seat 64, enlarged flow cavity 68, outlet channel 62, and outlet port 52), has caused the negative pressure differential between inlet port 50 and the outlet port 52 to increase. However, because the valve disk 66 is now in a closed position, thereby preventing the flow of fluid from the pressurized fluid source 16 to the aspiration flow path 46, the negative pressure differential between inlet port 50 and the outlet port 52 decreases until it reaches the negative activation pressure differential, causing the opposing force applied to the valve disk 66 by the fluid in the inlet channel 60 to rise to a level that overcomes the biasing force applied by the restoring spring 70 to the valve disk 66. As a result, the valve disk 66 is displaced from the closed position back to the open position (see **Fig. 8B**). The valve disk 66 continues to alternately be displaced between the closed position (see **Fig. 8A**) and the open position (see **Fig. 8B**) in this manner until the clog is removed from the aspiration conduit 24 of the aspiration catheter 12 or otherwise the anomaly in the aspiration circuit of the system 10 is resolved.

The frequency at which the valve disk 66 oscillates depends on the mass of the valve disk 66 (the frequency of the oscillation decreases as the mass of the valve disk 66 increases), the spring constant of the restoring spring 70 (the frequency of the oscillation increases as the spring constant of the restoring spring 70 increases), and the length of the valve seat 64 (the frequency of the oscillation increases as the length of the valve seat 64 decreases), as well as the dampening effect of the friction between the valve seat 64 and the valve disk 66 and the dynamic forces of the fluid flowing from the inlet channel 60, through the valve seat 64, and into the enlarged flow cavity 68, on the valve disk 66 (the frequency of the oscillation decreases as the dampening effect increases). Thus, the frequency at which the valve disk 66 oscillates (i.e., the resonance of the passive pressure oscillation assembly 44a) may be selected by judicially selecting the mass of the valve disk 66, spring constant of the restoring spring 70, length of the valve seat 64, and pre-compression length of the spring 70, with due regard to the dampening effect that the friction between the valve seat 64 and the valve disk 66 and the fluid flow associated pressure drop from the inlet channel 60, through the valve seat 64, and into the enlarged flow cavity 68, has on the valve disk 66. Such dampening effect, itself, may be adjusted by varying the designed sizes and geometries of the inlet port 50, outlet port 52, inlet channel 60, and outlet channel 62.

In response to removal of the clog in the aspiration conduit 24 of the aspiration catheter 12, or otherwise the resolution of the anomaly in the aspiration circuit of the system 10, the absolute pressure in the aspiration flow path 46 increases to a level that creates a negative cessation pressure differential between the inlet port 50 (and thus the inlet channel 60) and the outlet port 52 (and thus the enlarged flow cavity 68), which prevents the fluid in the inlet channel 60 from applying an opposing force to the valve disk 66 that overcomes the biasing force applied by the restoring spring 70 to the valve disk 66. That is, when the valve disk 66 is in the closed position, the negative pressure differential between inlet port 50 and the outlet port 52 will never drop below the negative activation pressure differential due to the free flow condition of the aspiration flow path 46. As a result, the biasing force applied by the restoring spring 70 maintains the valve disk 66 in the closed position. At this point, the passive pressure oscillation assembly 44a has been triggered to switch from the oscillatory mode back to the normal mode.

It should be noted that the passive pressure oscillation assembly 44a illustrated in **Figs. 8A** and **8B** topologically comprises a pressure actuated valve 54 and a fluid resonator 56 (shown in **Fig. 5**) that are mechanically coupled to each other. That is, the valve seat 64 and movable valve disk 66 form the pressure actuated valve 54, whereas the valve disk 66, enlarged flow cavity 68, and restoring spring 70 form the fluid resonator 56, with the pressure actuated valve 54 and fluid resonator 56 being mechanically coupled to each other via the valve disk 66. In this example, because the valve disk 66 forms a portion of both the pressure actuated valve 54 and the fluid resonator 56, the negative activation and cessation pressure differentials and the resonance frequency must be designed with due regard to each other, and thus, may not be able to be independently optimized, although the resulting design of the passive pressure oscillation assembly 44a may be mechanically simple.

Referring now to **Figs. 9A** and **9B****,** another example of a passive pressure oscillation assembly 44b will be described. The passive pressure oscillation assembly 44b is similar to the passive pressure oscillation assembly 44a illustrated in **Figs. 8A** and **8B****,** with the exception that the passive pressure oscillation assembly 44b comprises a long valve seal relative to the movable valve element in which it interacts, such that its resonant frequency is much slower than that of the passive pressure oscillation assembly 44a.

In particular, passive pressure oscillation assembly 44b comprises an inlet channel 80 fluidly coupled to the pressurized fluid source 16 via the inlet port 50, and an outlet channel 82 fluidly coupled to the aspiration flow path 46 via the outlet port 52. The passive pressure oscillation assembly 44b further comprises a valve seal in the form of a valve cylinder 84 fluidly coupled to the inlet port 50 via the inlet channel 80, a movable valve element in the form of a valve disk 86 operatively associated with the valve cylinder 84, and an enlarged flow cavity 88 fluidly coupled between the valve cylinder 84 and the aspiration flow path 46 via the outlet channel 62 and outlet port 52. The valve disk 86 is configured for being alternately displaced between a closed position to seal within the valve cylinder 84 (see **Fig. 9A**), and an open position residing within the enlarged flow cavity 88 (see **Fig. 9B**). The passive pressure oscillation assembly 44b further comprises a restoring spring 90 disposed in a spring cavity 92 between the enlarged flow cavity 88 and the outlet channel 82, and mechanically coupled to the valve disk 86 via a boss 94 affixed to the valve disk 86 for applying a biasing force to the valve disk 86 in a manner that maintains the valve disk 86 into the closed position within the valve cylinder 84 until the passive pressure oscillation assembly 44b is triggered to switch from the normal mode to the oscillatory mode.

The valve disk 86 and valve cylinder 84 have the same geometric profile (in this case, a cylindrical in nature), such that the valve disk 86, when in the closed position within the valve cylinder 84 (see **Fig. 9A**), seals against the valve cylinder 84 to prevent the flow of fluid originating from the pressurized fluid source 16 (in this case, fluid that has been introduced into the inlet channel 80 via the inlet port 50) into the enlarged flow cavity 88. The enlarged flow cavity 88 has a geometric profile that is larger than the geometric profile of the valve disk 86, such that the valve disk 86, when in the open position outside of the valve cylinder 84 and within the enlarged flow cavity 88 (see **Fig. 9B**), allows the flow of fluid originating from pressurized fluid source 16 (in this case, fluid that has been introduced into the inlet channel 80 via the inlet port 50), into the enlarged flow cavity 88, through the outlet channel 82, and into the aspiration flow path 46 via the outlet port 52.

In response to a clog in the aspiration conduit 24 of the aspiration catheter 12, or otherwise the occurrence of an anomaly in the aspiration circuit of the system 10, a no-flow or low-flow condition occurs in the aspiration flow path 46, and as a result, the absolute pressure in the aspiration flow path 46 drops to a level that creates a negative activation pressure differential between the inlet port 50 (and thus the valve cylinder 84) and the outlet port 52 (and thus the enlarged flow cavity 88), which causes the fluid in the inlet channel 80 to apply an opposing force to the valve disk 86 that overcomes the biasing force applied by the restoring spring 90 to the valve disk 86. As a result, the valve disk 86 is displaced from the closed position (see **Fig. 9A**) to the open position (see **Fig. 9B**). The negative activation pressure differential of the passive pressure oscillation assembly 44b will be based on the area of the valve disk 86 exposed to the fluid in the valve cylinder 84 (the negative activation pressure differential will decrease in proportion to the exposed area of the valve disk 86) and the spring constant of the restoring spring 90 (the negative action pressure differential will increase in proportion to the spring constant of the restoring spring 90). Thus, the negative activation pressure differential of the passive pressure oscillation assembly 44b may be selected by judicially selecting the exposed area of the valve disk 86 and the spring constant of the restoring spring 90.

The passive pressure oscillation assembly 44b is designed in such a manner that, once the valve disk 86 is displaced from the closed position to the open position (i.e., the valve "cracks"), the passive pressure oscillation assembly 44b resonates (i.e., the valve disk 86 alternately switches (oscillates) between the closed position and the open position. At this point, the passive pressure oscillation assembly 44b has been triggered to switch from the normal mode to the oscillatory mode.

In particular, the biasing force applied by the restoring spring 90 to the valve disk 86, the opposing force applied by the fluid in the valve cylinder 84, and the mass of the valve disk 86 are selected, such that the valve disk 86 oscillates between the closed position and the open position at a predetermined frequency (e.g., in the range of 0.2Hz-10Hz).

That is, when the valve disk 86 fully reaches the open position, the opposing force applied to the valve disk 86 by the fluid flowing from the inlet channel 80, through the valve cylinder 84, and into the enlarged flow cavity 88, drops to a level, such that the biasing force applied by the restoring spring 90 overcomes the opposing fluid force applied to the valve disk 86 and the momentum of the valve disk 86. As a result, the valve disk 86 is displaced from the open position back into the closed position within the valve cylinder 84 (see **Fig. 9A**). At this point, the momentary flow of fluid from the pressurized fluid source 16 into the aspiration flow path 46 (via the inlet port 50, inlet channel 80, valve cylinder 84, enlarged flow cavity 88, spring cavity 92, outlet channel 82, and outlet port 52), has caused the negative pressure differential between inlet port 50 and the outlet port 52 to increase. However, because the valve disk 86 is now in a closed position, thereby preventing the flow of fluid from the pressurized fluid source 16 to the aspiration flow path 46, the negative pressure differential between inlet port 50 and the outlet port 52 decreases until it reaches the negative activation pressure differential, causing the opposing force applied to the valve disk 86 by the fluid in the valve cylinder 84 to rise to a level that overcomes the biasing force applied by the restoring spring 90 to the valve disk 86. As a result, the valve disk 86 is again displaced from the closed position to the open position (see **Fig. 9B**). The valve disk 86 continues to alternately be displaced between the closed position (see **Fig. 9A**) and the open position (see **Fig. 9B**) in this manner until the clog is removed from the aspiration catheter 12 or otherwise the anomaly in the aspiration circuit of the system 10 is resolved.

The frequency at which the valve disk 86 oscillates depends on the mass of the valve disk 86 (the frequency of the oscillation decreases as the mass of the valve disk 86 increases), the spring constant of the restoring spring 90 (the frequency of the oscillation increases as the spring constant of the restoring spring 90 increases), and the length of the valve cylinder 84 (the frequency of the oscillation increases as the length of the valve seat 64 decreases), as well as the dampening effect of the friction between the valve cylinder 84 and the valve disk 86 and the dynamic forces of the fluid flowing from the inlet channel 60, through the valve cylinder 84, and into the enlarged flow cavity 88, on the valve disk 86 (the frequency of the oscillation decreases as the dampening effect increases). Thus, frequency at which the valve disk 86 oscillates (i.e., the resonance of the passive pressure oscillation assembly 44b) may be selected by judicially selecting the mass of the valve disk 86 and the spring constant of the restoring spring 90, length of the valve cylinder 84, and pre-compression length of the spring 90, with due regard to the dampening effect that the friction between the valve cylinder 84 and the valve disk 86 and the fluid flowing from the inlet channel 80, through the valve cylinder 84, and into the enlarged flow cavity 88, has on the valve disk 86. Such dampening effect, itself, may be adjusted by varying the designed sizes and geometries of the inlet port 50, outlet port 52, inlet channel 80, and outlet channel 82.

In response to removal of the clog in the aspiration conduit 24 of the aspiration catheter 12, or otherwise the resolution of the anomaly in the aspiration circuit of the system 10, the absolute pressure in the aspiration flow path 46 increases to a level that creates a negative activation pressure differential between the inlet port 50 (and thus the valve cylinder 84) and the outlet port 52 (and thus the enlarged flow cavity 88), which prevents the fluid in the inlet channel 80 from applying an opposing force to the valve disk 86 that overcomes the biasing force applied by the restoring spring 90 to the valve disk 86. That is, when the movable valve cylinder 86 is in the closed position, the negative pressure differential between inlet port 50 and the outlet port 52 will never drop below the negative activation pressure differential due to the free flow condition of the aspiration flow path 46. As a result, the biasing force applied by the restoring spring 90 maintains the valve disk 86 in the closed position. At this point, the passive pressure oscillation assembly 44b has been triggered to switch from the oscillatory mode to the normal mode.

It should be noted that the passive pressure oscillation assembly 44b illustrated in **Figs. 9A** and **9B** topologically comprises a pressure actuated valve 54 and a fluid resonator 56 (shown in **Fig. 5**) that are mechanically coupled to each other. That is, the valve cylinder 84 and valve disk 86 form the pressure actuated valve 54, whereas the valve disk 86, enlarged flow cavity 88, and restoring spring 90 form the fluid resonator 56, with the pressure actuated valve 54 and fluid resonator 56 being mechanically coupled to each other via the valve disk 86. In this example, because the valve disk 86 forms a portion of both the pressure actuated valve 54 and the fluid resonator 56, the negative activation and cessation pressure differentials and the resonance frequency must be designed with due regard to each other, and thus, may not be able to be independently optimized, although the resulting design of the passive pressure oscillation assembly 44b may be mechanically simple.

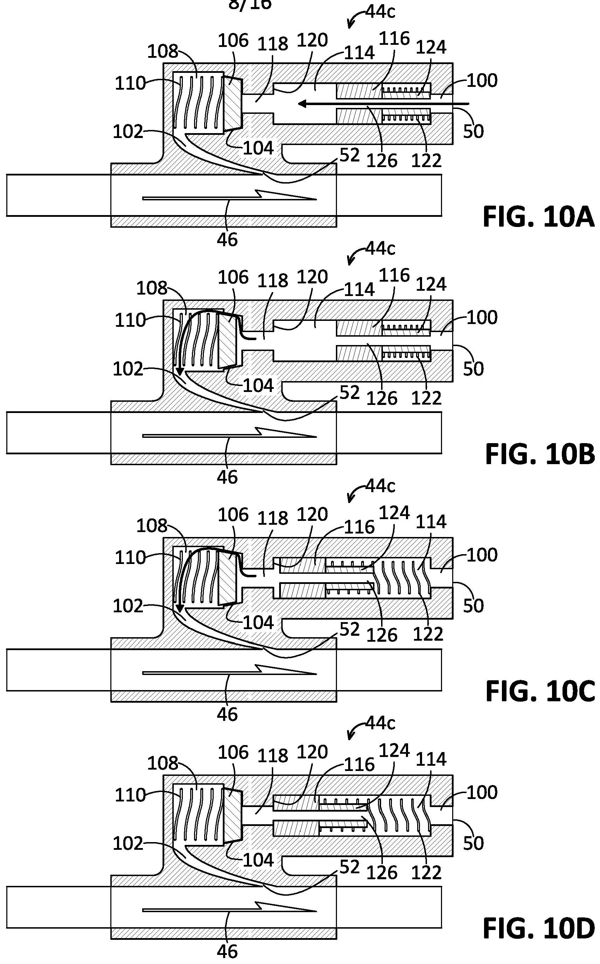

Referring now to **Figs. 10A-10D****,** still another example of a passive pressure oscillation assembly 44c will be described. The passive pressure oscillation assembly 44c is similar to the passive pressure oscillation assembly 44a illustrated in **Figs. 6A** and **6B,** with the exception that the passive pressure oscillation assembly 44c comprises an additional oscillation enhancement mechanism that ensures that the passive pressure oscillation assembly 44c remains in the oscillatory mode as long as the clog in the aspiration conduit 24 of the aspiration catheter 12 remains, or otherwise the anomaly in the aspiration circuit of the system 10 is not resolved.

The passive pressure oscillation assembly 44c comprises an inlet channel 100 fluidly coupled to the pressurized fluid source 16 via the inlet port 50, and an outlet channel 102 fluidly coupled to the aspiration flow path 46 via the outlet port 52. The passive pressure oscillation assembly 44c further comprises a valve seal in the form of a seat 104, a movable valve element in the form of a valve disk 106 operably associated with the valve seat 104, and an enlarged flow cavity 108 that fluidly couples the valve seat 104 to the aspiration flow path 46 via the outlet channel 102 and outlet port 52. The valve disk 106 is configured for being alternately displaced between a closed position to seal against the valve seat 104, and in this case, within the valve seat 104 (see **Figs. 10A** and **10D**), and an open position away from the valve seat 104, and in this case outside of the valve seat 104 (see **Figs. 10B** and **10C****).** The passive pressure oscillation assembly 44c further comprises a restoring spring 110 disposed in the enlarged flow cavity 108 and mechanically coupled to the valve disk 106 for applying a biasing force to the valve disk 106 in a manner that maintains the valve disk 106 into the closed position within the valve seat 104 until the passive pressure oscillation assembly 44c is triggered to switch from the normal mode to the oscillatory mode.

The passive pressure oscillation assembly 44c further comprises a plunger cavity 114, a plunger head 116 slidably disposed within the plunger cavity 114, a reduced profile center cavity 118, a plunger stop 120 disposed between the plunger cavity 114 and the reduced profile center cavity 118, and another restoring spring 122 mechanically coupled to the plunger head 116 via a boss 124 affixed to the plunger head 116 for applying a biasing force to the plunger head 116 to maintain the plunger head 116 away from the plunger stop 120. In the illustrated example, the profile of the reduced profile center cavity 118 is smaller than the profile of the plunger cavity 114, such that the plunger stop 120 is formed by the wall of the plunger cavity 114 adjacent the reduced profile center cavity 118. The plunger head 116 has a fluid pressure equalization channel 126 extending through the plunger head 116. The plunger cavity 114 is fluidly coupled between the valve seat 104 and the plunger cavity 114, such that the valve seat 104 is always in fluid communication with the inlet port 50 via the fluid pressure equalization channel 126 extending through the plunger head 116, and fluid originating from the pressurized fluid source 16 can flow into the reduced profile center cavity 118. Thus, the fluid pressure equalization channel 126 extending through the plunger head 116 serves to equalize the pressure between the pressurized fluid source 16 and the reduced profile center cavity 118.

The valve disk 106 and valve seat 104 have the same geometric profile (in this case, a flattened trapezoidal shape cross-section), such that the valve disk 106, when in the closed position within the valve seat 104 (see **Figs. 10A** and **10D**), prevents the flow of fluid originating from the pressurized fluid source 16 (in this case, fluid that has been introduced into the reduced profile center cavity 118 from the inlet channel 100 via the inlet port 50, and through the fluid pressure equalization channel 126 of the plunger head 116) into the enlarged flow cavity 108. The enlarged flow cavity 108 has a geometric profile that is larger than the geometric profile of the valve disk 106, such that the valve disk 106, when in the open position outside of the valve seat 104 and inside the enlarged flow cavity 108 (see **Figs. 10B** and **10C**), allows the flow of fluid originating from pressurized fluid source 16 (in this case, fluid that has been introduced into plunger cavity 114 from the inlet port 50 and the inlet channel 100 via the fluid pressure equalization channel 126), into the enlarged flow cavity 108, through the outlet channel 102, and into the aspiration flow path 46 via the outlet port 52. The plunger head 116 and plunger cavity 114 have the same geometric profile (in this case, a cylindrical in nature), such fluid from the pressurized fluid source 16 can only enter the reduced profile center cavity 118 via the fluid pressure equalization channel 126 of the plunger head 116.

In response to a clog in the aspiration conduit 24 of the aspiration catheter 12, or otherwise the occurrence of an anomaly in the aspiration circuit of the system 10, a no-flow or low-flow condition occurs in the aspiration flow path 46, and as a result, the absolute pressure in the aspiration flow path 46 drops to a level that creates a negative activation pressure differential between the inlet port 50 (and thus the reduced profile center cavity 118) and the outlet port 52 (and thus the enlarged flow cavity 108), which causes the fluid in the plunger cavity 114, and thus reduced profile center cavity 118, to apply an opposing force to the valve disk 106 that overcomes the biasing force applied by the restoring spring 110 to the valve disk 106. As a result, the valve disk 106 is displaced from the closed position (see **Fig.** 10A) to the open position (see **Fig. 10B**). The negative activation pressure differential of the passive pressure oscillation assembly 44c will be based on the area of the valve disk 106 exposed to the fluid in the inlet channel 100 (the negative activation pressure differential will decrease in proportion to the exposed area of the valve disk 106) and the spring constant of the restoring spring 110 (the negative action pressure differential will increase in proportion to the spring constant of the restoring spring 110). Thus, the negative activation pressure differential of the passive pressure oscillation assembly 44c may be selected by judicially selecting the exposed area of the valve disk 106 and the spring constant of the restoring spring 110.

The passive pressure oscillation assembly 44c is designed in such a manner that, once the valve disk 106 is displaced from the closed position to the open position (i.e., the valve "cracks"), the passive pressure oscillation assembly 44c resonates (i.e., the valve disk 106 alternately switches (oscillates) between the closed position and the open position. At this point, the passive pressure oscillation assembly 44c has been triggered to switch from the normal mode to the oscillatory mode.

In particular, the biasing force applied by the restoring spring 110 to the valve disk 106, the opposing force applied by the fluid in the reduced profile center cavity 118 to the valve disk 106, and mass of the valve disk 106 are selected, such that the valve disk 106 oscillates between the closed position and the open position at a predetermined frequency. Furthermore, dynamic displacement of the plunger head 116 within the plunger cavity 114 ensures that the valve disk 106 does not get stuck in the open position as the fluid flowing from the reduced profile center cavity 118 into the enlarged flow cavity 108 applies a force to the valve disk 106.

In particular, as the valve disk 106 is displaced from the closed position to the open position (see **Fig. 10C**), fluid flows from the plunger cavity 114 in front of the plunger head 116, through the reduced profile center cavity 118, through the valve seat 104, and into the enlarged flow cavity 108, causing the plunger head 116 to be displaced within the plunger cavity 114 towards the reduced profile center cavity 118 until the plunger head 116 abuts the plunger stop 120, and additional fluid to flow from the pressurized fluid source 16 into the plunger cavity 114 behind the plunger head 116 via the inlet port 50 and inlet channel 100. Once the plunger head 116 abuts the plunger stop 120, the flow of fluid from the reduced profile center cavity 118, through the valve seat 104, and into the enlarged flow cavity 108 is greatly diminished, limited to the flow of fluid through the fluid pressure equalization channel 126 through the plunger head 116. Thus, the opposing force applied to the valve disk 106 by the fluid flowing from the reduced profile center cavity 118, through the valve seat 104, and into the enlarged flow cavity 108, drops to a level, such that the biasing force applied by the restoring spring 110 overcomes the opposing fluid force applied to the valve disk 106 and the momentum of the valve disk 106. As a result, the valve disk 106 is displaced from the open position back into the closed position within the valve seat 104 (see **Fig. 10D**). The fluid pressure between the reduced profile center cavity 118 and the plunger cavity 114 equalizes via the fluid pressure equalization channel 126 through the plunger head 116, thereby dropping the opposing force applied to the plunger head 116 by the fluid in the plunger cavity 114 to a level, such that the biasing force applied by the restoring spring 122 overcomes the opposing fluid force applied to the plunger head 116 and the momentum of the plunger head 116. As a result, the plunger head 116 is displaced within the plunger cavity 114 away from the plunger stop 120, and returns to its neutral position (see **Fig. 10A**). In this manner, in contrast to the passive pressure oscillation assembly 44a illustrated in **Figs. 8A-8B****,** as well as the passive pressure oscillation assembly 44a illustrated in **Figs. 9A-9B****,** where fluid flows through the valve seat unimpeded, which under certain circumstances, may cause the valve disk to remain open, thereby preventing oscillation of the valve seat between the closed and open positions, the action of the plunger head 116 within the plunger cavity 114 prevents the valve disk 106 from "sticking" in the open position by greatly diminishing the flow of fluid through the valve seat 104 that might otherwise prevent the valve disk 106 to revert back to its closed position within the valve seat 104.

The frequency at which the valve disk 106 oscillates depends on the frequency at which the plunger head 116 oscillates within the plunger cavity 114, which in turn, depends on the mass of the plunger head 116 (the frequency of the oscillation decreases as the mass of the plunger head 116 increases), the spring constant of the restoring spring 122 (the frequency of the oscillation increases as the spring constant of the restoring spring 122 increases), the diameter of the equalization channel 126, and the dampening effect of the friction between the plunger cavity 114 and the plunger head 116, as well as dynamic forces of the fluid within the plunger cavity 114, including the fluid flowing through the channel fluid pressure equalization channel 126 of the plunger head 116 during equalization of the fluid pressure in the plunger cavity 114 (the frequency of the oscillation decreases as the dampening effect increases). Thus, frequency at which the valve disk 106 oscillates (i.e., the resonance of the passive pressure oscillation assembly 44c) may be selected by judicially selecting the mass of the plunger head 116 and the spring constant of the restoring spring 122, and the diameter of the equalization channel 126, with due regard to the dampening effect that the friction between the plunger cavity 114 and the plunger head 116, and the dynamics of the fluid within the reduced profile center cavity 118, have on the plunger head 116. Such dampening effect, itself, may be adjusted by varying the designed size of the inlet port 50, outlet port 52, inlet channel 100, and outlet channel 102.

In response to removal of the clog in the aspiration conduit 24 of the aspiration catheter 12, or otherwise the resolution of the anomaly in the aspiration circuit of the system 10, the absolute pressure in the aspiration flow path 46 increases to a level that creates a negative cessation pressure differential between the inlet port 50 (and thus the inlet channel 100) and the outlet port 52 (and thus the enlarged flow cavity 108), which prevents the fluid in the reduced profile center cavity 118 from applying an opposing force to the valve disk 106 that overcomes the biasing force applied by the spring 110 to the valve disk 106. That is, when the valve disk 106 is in the closed position, the negative pressure differential between inlet port 50 and the outlet port 52 will never drop below the negative activation pressure differential due to the free flow condition of the aspiration flow path 46. As a result, the biasing force applied by the restoring spring 110 maintains the valve disk 106 in the closed position. At this point, the passive pressure oscillation assembly 44c has been triggered to switch from the oscillatory mode to the normal mode.

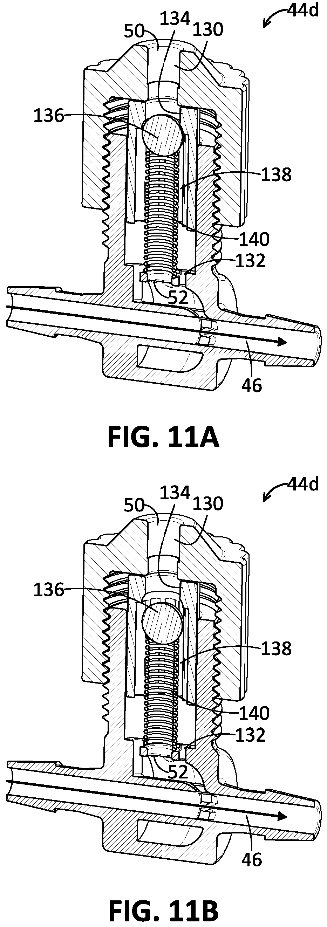

Although the movable valve elements in the passive pressure oscillation assemblies 44a-44c illustrated in **Figs. 8-10** have been described as being valve disks, it should be appreciated that the movable valve elements may have any suitable form that can be operatively associated with a corresponding valve seal for alternately allowing and preventing the flow of fluid originating from the pressurized fluid source 16 therethrough. For example, with reference to **Figs. 11A** and **11B****,** an alternative example of a passive pressure oscillation assembly 44d is similar to the passive pressure oscillation assembly 44a of **Figs. 8A-8B****,** with the exception that the movable valve element takes the form of a ball.

In particular, the passive pressure oscillation assembly 44d comprises a comprises an inlet channel 130 fluidly coupled to the pressurized fluid source 16 via the inlet port 50, and an outlet channel 132 fluidly coupled to the aspiration flow path 46 via the outlet port 52. The passive pressure oscillation assembly 44d further comprises a valve seal in the form of a seat 134 fluidly coupled to the inlet port 50 via the inlet channel 130, a movable valve element in the form of a valve ball 136 operably associated with the valve seat 134, and an enlarged flow cavity 138 fluidly coupled between the valve seat 134 and the aspiration flow path 46 via the outlet channel 132 and outlet port 52. The valve ball 136 is configured for being alternately displaced between a closed position to seal against the valve seat 134 (see **Fig. 11A****),** and an open position away from the valve seat 64, and in this case outside of the valve seat 64 (see **Fig. 11B**). The passive pressure oscillation assembly 44d further comprises a spring 140 affixed within the enlarged flow cavity 138 and mechanically coupled to the valve ball 136 for applying a biasing force to the valve ball 136 in a manner that maintains the valve ball 136 in the closed position against valve seat 134 until the passive pressure oscillation assembly 44d is triggered to switch from the normal mode to the oscillatory mode, as will be described in further detail below.

The surface of the valve seat 134 that contacts the valve ball 136 preferably has a spherical profile, such that the valve ball 136, when in the closed position against the valve seat 134 (see **Fig. 11A**), seals against the valve seat 134 to prevent the flow of fluid originating from the pressurized fluid source 16 (in this case, fluid that has been introduced into the inlet channel 130 via the inlet port 50) into the enlarged flow cavity 138. The enlarged flow cavity 138 has a geometric profile that is larger than the geometric profile of the valve ball 136, such that the valve ball 136, when in the open position away from the valve seat 134 and within the enlarged flow cavity 138 (see **Fig. 11B**), allows the flow of fluid originating from pressurized fluid source 16 (in this case, fluid that has been introduced into the inlet channel 130 via the inlet port 50), into the enlarged flow cavity 138, through the outlet channel 132, and into the aspiration flow path 46 via the outlet port 52.

In response to a clog in the aspiration conduit 24 of the aspiration catheter 12, or otherwise the occurrence of an anomaly in the aspiration circuit of the system 10, a no-flow or low-flow condition occurs in the aspiration flow path 46, and as a result, the absolute pressure in the aspiration flow path 46 drops to a level that creates a negative activation pressure differential between the inlet port 50 (and thus the inlet channel 130) and the outlet port 52 (and thus the enlarged flow cavity 138), which causes the fluid in the inlet channel 130 to apply an opposing force to the valve ball 136 that overcomes the biasing force applied by the spring 140 to the valve ball 136. As a result, the valve ball 136 is displaced from the closed position (see **Fig. 11A**) to the open position (see **Fig. 11B**). The negative activation pressure differential of the passive pressure oscillation assembly 44d will be based on the area of the valve ball 136 exposed to the fluid in the inlet channel 130 (the negative activation pressure differential will decrease in proportion to the exposed area of the valve ball 136) and the spring constant of the spring 140 (the negative action pressure differential will increase in proportion to the spring constant of the spring 140). Thus, the negative activation pressure differential of the passive pressure oscillation assembly 44d may be selected by judicially selecting the exposed area of the valve ball 136 and the spring constant of the spring 140.

The passive pressure oscillation assembly 44d is designed in such a manner that, once the valve ball 136 is displaced from the closed position to the open position (i.e., the valve "cracks"), the passive pressure oscillation assembly 44d resonates (i.e., the valve ball 136 alternately switches (oscillates) between the closed position and the open position. At this point, the passive pressure oscillation assembly 44d has been triggered to switch from the normal mode to the oscillatory mode.

In particular, the biasing force applied by the spring 140 to the valve ball 136, the opposing force applied by the fluid in the inlet channel 130 to the valve ball 136, and the mass of the valve ball 136 are selected, such that the valve ball 136 oscillates between the closed position and the open position at a predetermined frequency (e.g., 0.2Hz-10Hz).

That is, when the valve ball 136 initially reaches the fully open position, the opposing force applied to the valve ball 136 by the fluid flowing from the inlet channel 60, through the valve seat 134, and into the enlarged flow cavity 138, drops to a level, such that the biasing force applied by the spring 140 overcomes the opposing fluid force applied to the valve ball 136 and the momentum of the valve ball 136. As a result, the valve ball 136 is displaced from the open position back into the closed position within the valve seat 134 (see **Fig. 11A**). At this point, the momentary flow of fluid from the pressurized fluid source 16 into the aspiration flow path 46 (via the inlet port 50, inlet channel 130, valve seat 134, enlarged flow cavity 138, outlet channel 132, and outlet port 52), has caused the negative pressure differential between inlet port 50 and the outlet port 52 to increase. However, because the valve ball 136 is now in a closed position, thereby preventing the flow of fluid from the pressurized fluid source 16 to the aspiration flow path 46, the negative pressure differential between inlet port 50 and the outlet port 52 decreases until it reaches the negative activation pressure differential, causing the opposing force applied to the valve ball 136 by the fluid in the inlet channel 60 to rise to a level that overcomes the biasing force applied by the spring 140 to the valve ball 136. As a result, the valve ball 136 is displaced from the closed position back to the open position (see **Fig. 11B**). The valve ball 136 continues to alternately be displaced between the closed position (see **Fig. 11A**) and the open position (see **Fig. 11B**) in this manner until the clog is removed from the aspiration conduit 24 of the aspiration catheter 12 or otherwise the anomaly in the aspiration circuit of the system 10 is resolved.

The frequency at which the valve ball 136 oscillates depends on the mass of the valve ball 136 (the frequency of the oscillation decreases as the mass of the valve ball 136 increases), and the spring constant of the spring 140 (the frequency of the oscillation increases as the spring constant of the spring 140 increases), as well as the dampening effect of the dynamic forces of the fluid flowing from the inlet channel 130, through the valve seat 134, and into the enlarged flow cavity 138, on the valve ball 136 (the frequency of the oscillation decreases as the dampening effect increases). Thus, the frequency at which the valve ball 136 oscillates (i.e., the resonance of the passive pressure oscillation assembly 44a) may be selected by judicially selecting the mass of the valve ball 136, spring constant of the spring 140, length of the valve seat 134, and pre-compression length of the spring 140, with due regard to the dampening effect that the fluid flowing from the inlet channel 130, through the valve seat 134, and into the enlarged flow cavity 138, has on the valve ball 136. Such dampening effect, itself, may be adjusted by varying the designed sizes and geometries of the inlet port 50, outlet port 52, inlet channel 130, and outlet channel 132.

In response to removal of the clog in the aspiration conduit 24 of the aspiration catheter 12, or otherwise the resolution of the anomaly in the aspiration circuit of the system 10, the absolute pressure in the aspiration flow path 46 increases to a level that creates a negative cessation pressure differential between the inlet port 50 (and thus the inlet channel 130) and the outlet port 52 (and thus the enlarged flow cavity 138), which prevents the fluid in the inlet channel 130 from applying an opposing force to the valve ball 136 that overcomes the biasing force applied by the spring 140 to the valve ball 136. That is, when the valve ball 136 is in the closed position, the negative pressure differential between inlet port 50 and the outlet port 52 will never drop below the negative activation pressure differential due to the free flow condition of the aspiration flow path 46. As a result, the biasing force applied by the spring 140 maintains the valve ball 136 in the closed position. At this point, the passive pressure oscillation assembly 44d has been triggered to switch from the oscillatory mode back to the normal mode.

It should be noted that the passive pressure oscillation assembly 44d illustrated in **Figs. 11A** and **11B** topologically comprises a pressure actuated valve 54 and a fluid resonator 56 (shown in **Fig. 5**) that are mechanically coupled to each other. That is, the valve seat 134 and movable valve ball 136 form the pressure actuated valve 54, whereas the valve ball 136, enlarged flow cavity 138, and spring 140 form the fluid resonator 56, with the pressure actuated valve 54 and fluid resonator 56 being mechanically coupled to each other via the valve ball 136. In this example, because the valve ball 136 forms a portion of both the pressure actuated valve 54 and the fluid resonator 56, the negative activation and cessation pressure differentials and the resonance frequency must be designed with due regard to each other, and thus, may not be able to be independently optimized, although the resulting design of the passive pressure oscillation assembly 44d may be mechanically simple.

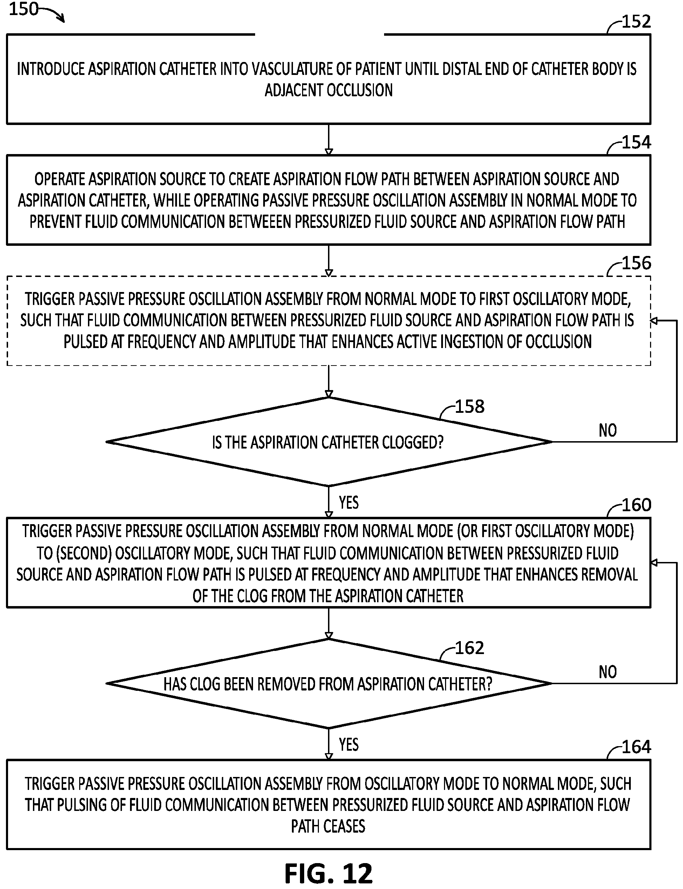

Referring now to **Fig. 12****,** one method (not part of the invention) 150 of operating the aspiration system 10 to aspirate the occlusion 2 from the vasculature 1 of a patient will be described. The method (not part of the invention) 150 comprises introducing the aspiration catheter 12 into the vasculature 1 of the patient until the distal end 30 of the catheter body 22 is adjacent the occlusion 2 (step 152). Next, the aspiration source 14 is operated to create an aspiration flow path 46 between the aspiration catheter 12 and the aspiration source 14 to actively ingest the occlusion 2, while operating the passive pressure oscillation assembly 44 in the normal mode to prevent fluid communication between the pressurized fluid source 16 and the aspiration flow path 46 (step 154). Thus, aspiration of the occlusion 2 is performed as efficiently as possible at this point.

Optionally, the passive pressure oscillation assembly 44 is triggered to switch from the normal mode to a first oscillatory mode in response to active ingestion of the occlusion 2 (e.g., if the absolute pressure in the aspiration flow path 46 drops to a level that creates a first negative activation pressure differential between the pressurized fluid source 16 and the aspiration flow path 46 less than - 50kPa), such that fluid communication between the pressurized fluid source 16 and the aspiration flow path 46 is pulsed at a suitable amplitude and frequency that enhances active ingestion of the occlusion 2 (e.g., high frequency, low amplitude) (step 156). The high frequency, low amplitude pulsing of the fluid communication between the pressurized fluid source 16 and the aspiration flow path 46 may minimize disruption to the aspiration flow path 46, such that active ingestion of the occlusion 2 may be as efficient as possible.

Next, if a clog occurs in the aspiration conduit 24 of the aspiration catheter 12 (e.g., if the absolute pressure in the aspiration flow path 46 drops to a level that creates a second negative activation pressure differential between the pressurized fluid source 16 and the aspiration flow path 46 less than -55kPa) (step 158), the passive pressure oscillation assembly 44 is triggered to switch from the normal mode (or optionally the first oscillatory mode) to the (second) oscillatory mode, such that fluid communication between the pressurized fluid source 16 and the aspiration flow path 46 is pulsed at a suitable amplitude and frequency that enhances clearing of the clog (e.g., low frequency, high amplitude) (step 160). Optionally, the fluid communication between the pressurized fluid source 16 and the aspiration flow path 46 may be pulsed simultaneously at different frequencies. If a clog does not occur in the aspiration conduit 24 of the aspiration catheter 12 (e.g., if the absolute pressure in the aspiration flow path 46 does not drop to a level that creates a second negative activation pressure differential between the pressurized fluid source 44 and the aspiration flow path 46 less than -55kPa) (step 158), the passive pressure oscillation assembly 44 remains in the normal mode (or optionally in the first oscillatory mode) until the occlusion 2 is fully ingested.

If a clog does occur in the aspiration conduit 24 of the aspiration catheter 12, and such clog has been removed, or otherwise the anomaly in the aspiration circuit of the aspiration system 10 has been resolved (e.g., if the absolute pressure in the aspiration flow path 46 rises to a level that creates a negative cessation pressure differential between the pressurized fluid source 16 and the aspiration flow path 46 that is greater, and preferably 10kPa-25kPa greater, than the negative activation pressure differential) (step 162), the passive pressure oscillation assembly 44 is triggered to switch from the oscillatory mode to the normal mode, such that fluid communication between the pressurized fluid source 16 and the aspiration flow path 46 is again prevented, and the aspiration procedure continues (step 164). If a clog does occur in the aspiration conduit 24 of the aspiration catheter 12, and such clog has not been removed, or otherwise the anomaly in the aspiration circuit of the aspiration system 10 has not been resolved (e.g., if the absolute pressure in the aspiration flow path 46 does not rise to a level that creates a negative cessation pressure differential between the pressurized fluid source 44 and the aspiration flow path 46 that is greater, and preferably 10kPa-25kPa greater, than the negative activation pressure differential), the passive pressure oscillation assembly 44 remains in the (second) oscillator mode until the occlusion 2 has been removed, or otherwise the anomaly in the aspiration circuit of the aspiration system 10 has been resolved.

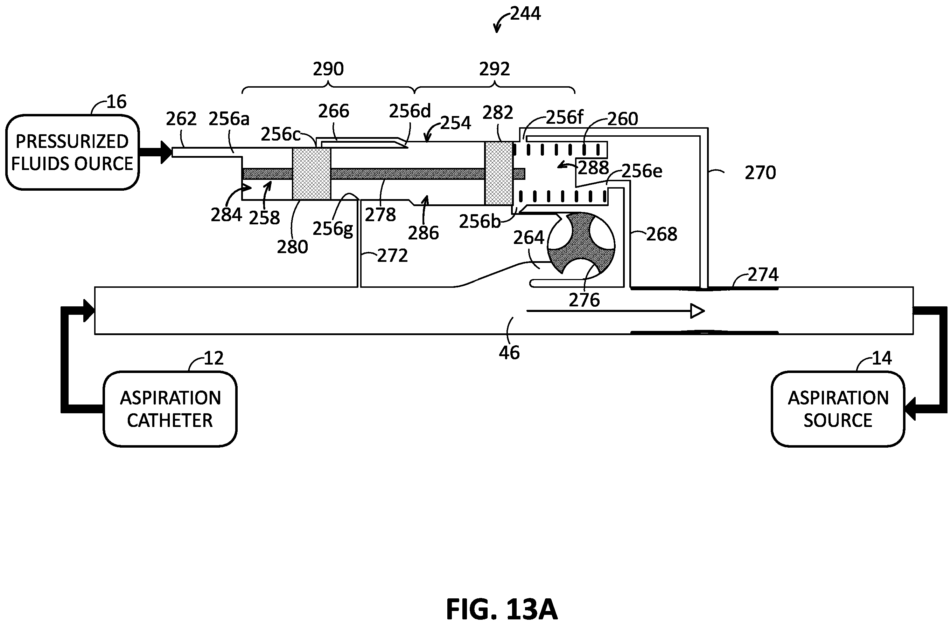

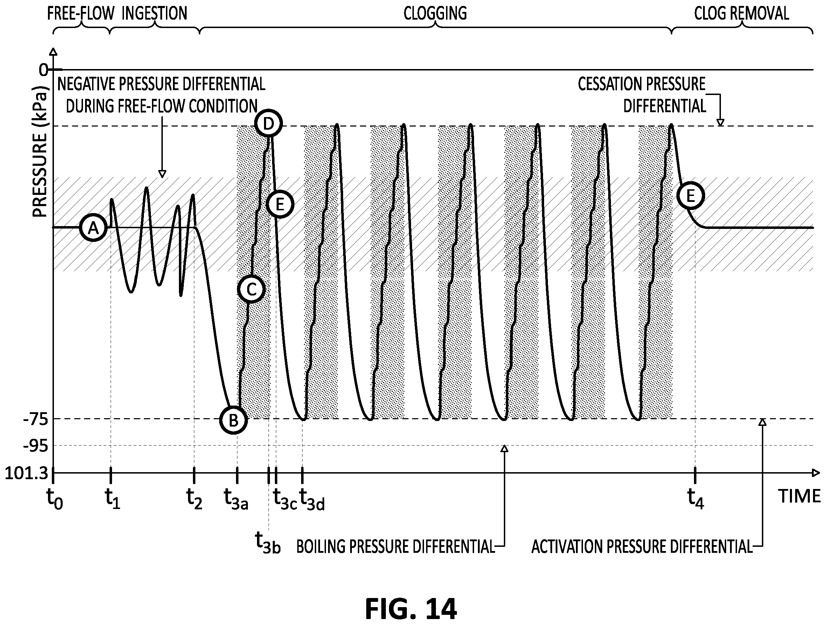

Although the cessation pressure differential of the passive pressure oscillation assembly 44, and the variations thereof, have been described as being below the negative activation pressure differential resulting from the free flow condition of the aspiration flow path 46, the cessation pressure differential of one advantageous embodiment of a passive pressure oscillation assembly 244 is above the negative activation pressure differential resulting from the free flow condition of the aspiration flow path 46, as illustrated in **Figs. 13A-13E** and **14.**

Similar to the passive pressure oscillation assembly 44, the passive pressure oscillation assembly 244 is configured for dynamically loading (i.e., rapidly changing the vacuum level) the aspiration conduit 24 of the aspiration catheter 12 (shown in **Fig. 1**), and in particular, cyclically loading the aspiration conduit 24 only during the no-flow or low-flow conditions. Like the passive pressure oscillation assembly 44, the passive pressure oscillation assembly 44 accomplishes this without user input and without the use of electronic sensors, may be made to be very compact, such that it can be fitted within manifold 20 will little additional bulk, and may be disabled simply by blocking the relief inlet 40.

Like the passive pressure oscillation assembly 44, the passive pressure oscillation assembly 244 is configured for being operated between a normal mode that prevents fluid communication along a relief path 48 between the pressurized fluid source 16 and the aspiration flow path 46, such that the absolute pressure in the aspiration flow path 46 remains relatively constant and is only acted upon by the aspiration source 14, and an oscillatory mode that pulses fluid communication along the relief path 48 between the pressurized fluid source 16 and the aspiration flow path 46, such that the absolute pressure in the aspiration flow path 46 oscillates within a range of predetermined frequencies. The passive pressure oscillation assembly 244 is configured for being triggered to switch from the normal mode to the oscillatory mode in response to a clog in the aspiration conduit 24 of the aspiration catheter 12 or otherwise a flow anomaly in the aspiration conduit of the system 10, and conversely, for being triggered to switch from the oscillatory mode to the normal mode in response to removal or clearance of the clog from the aspiration conduit 24 of the aspiration catheter 12 or otherwise resolution of the flow anomaly in the aspiration pcircuit of the system 10. In the illustrated embodiment, fluid communication pulsing between the pressurized fluid source 16 and the aspiration flow path 46 causes pressure pulses to propagate down the aspiration conduit 24 of the aspiration catheter 12 at one or more predetermined frequencies, and as will be described in further below, two predetermined frequencies. Simultaneously, fluid communication pulsing between the pressurized fluid source 16 and the aspiration flow path 46 causes fluid backflows to propagate down the aspiration conduit 24 of the aspiration catheter 12.

Unlike the passive pressure oscillation assembly 44, because the cessation pressure differential of the passive pressure oscillation assembly 244 is above a negative activation pressure differential resulting from the free flow condition of the aspiration flow path 46, the negative cessation pressure differential may be designed to be much greater than the negative activation pressure differential, thereby ultimately increasing the oscillation strength for dislodging the clog from the aspiration conduit 24 of the aspiration catheter 12. For example, whereas the previously described passive pressure oscillation assembly 44, and the variations thereof, may have a negative cessation pressure differential that is 10kPa-25kPa greater than the negative activation pressure differential, the passive pressure oscillation assembly 244 may have a negative cessation pressure differential that is 40kPa-90kPa greater than the negative activation pressure differential.

The passive pressure oscillation assembly 244 is also configured for being operating in a mixed frequency mode. In particular, the passive pressure oscillation assembly 244 can be operated in an oscillatory mode that pulses fluid communication between the pressurized fluid source 16 and the aspiration flow path 46 concurrently at a first frequency (e.g., in the range of 0.2Hz-10Hz) and at a second frequency different from the first frequency (e.g., in the range of 100Hz-400Hz). In this manner, the lower frequency oscillatory mode of the passive pressure oscillation assembly 244 may exert a relatively constant large force on the thrombus 2 in the aspiration conduit 24 of the aspiration catheter 12, while the high frequency oscillatory mode of the passive pressure oscillation assembly may exert a fluctuating small force on the thrombus 2, thereby reducing the friction between the thrombus 2 and the aspiration conduit 24.

The passive pressure oscillation assembly 244 comprises a plunger cavity 254 having a multitude of cavity ports 256 (including an inlet port 256a, outlet port 256b, bypass ports 256c, 256d, pressure tap port 256e, aspiration shutoff port 256f, and pressure equalization port 256g) all spaced from each other along a length of the plunger cavity 254, a plunger assembly 258 slidably disposed within the plunger cavity 254, a spring 260 configured for applying a biasing force to the plunger assembly 254, a multitude of channels, including an inlet channel 262, an outlet channel 264, a bypass channel 266, a pressure tap channel 268, an aspiration shutoff channel 270, and a pressure equalization channel 272, all conditionally in fluid communication with the plunger cavity 254 via the multitude of ports 256, a fluid-actuated aspiration shutoff valve 274 disposed within the aspiration flow path 46 between the aspiration source 14 and the pressure tap channel 268, and a fluid resonator 276 disposed in the outlet channel 262.

The plunger assembly 258 includes a rod 278, a first plunger head 280 affixed to the rod 278, and a second plunger head 282 affixed to the rod 278 in a spaced apart relationship with the first plunger head 280, thereby forming a front plunger cavity region 284 in front of the first plunger head 280, a center plunger cavity region 286 between the first plunger head 280 and the second plunger head 282, and a rear plunger cavity region 288 behind the second plunger head 282. As will be described in further detail below, when the passive pressure oscillation assembly 244 is operating in the oscillatory mode, the plunger assembly 258 is configured for being displaced within the plunger cavity 254 between an open position and a closed position to create low-frequency fluid pulses in the aspiration flow patch 46. Thus, the oscillatory mode of the passive pressure oscillation assembly 244 may pulse fluid communication between the pressurized fluid source 16 and the aspiration flow path 46 at a first frequency. For reasons that will be discussed in further detail below, the outer diameter of the second plunger head 282 is greater than the outer diameter of the first plunger head 280. The plunger cavity 254 has a first portion 290 having a first diameter for accommodating the first plunger head 280, and a second portion 292 having a second diameter greater than the first diameter for accommodating the second plunger head 282.

The inlet channel 262 is in fluid communication between the pressurized fluid source 16 and the front plunger cavity region 284 (via the inlet port 256a of the plunger cavity 254). The outlet channel 264 is conditionally in fluid communication between the center plunger cavity region 286 (via the outlet cavity port 256b of the plunger cavity 254) and the aspiration flow path 46. The bypass channel 266 is conditionally in fluid communication between the front plunger cavity region 284 (via the first bypass port 256c of the plunger cavity 254) and the center plunger cavity region 286 (via the second bypass port 256d of the plunger cavity 254). The pressure tap channel 268 is in fluid communication between the aspiration flow path 46 and the rear plunger cavity region 288 (via the pressure tap port 256e of the plunger cavity 254). The aspiration shutoff channel 270 is conditionally in fluid communication between the center plunger cavity region 286 (via the aspiration shutoff port 256f of the plunger cavity 254) and the fluid-actuated valve 274), and conditionally in fluid communication between the rear plunger cavity region 288 (via the aspiration shutoff port 256f of the plunger cavity 254) and the fluid-actuated valve 274). The pressure equalization channel 272 is conditionally in fluid communication between the aspiration flow path 46 and the center plunger cavity region 286 (via the pressure equalization port 256g of the plunger cavity 254).

When the passive pressure oscillation assembly 244 is operating in the oscillatory mode, the fluid resonator 276 is configured for resonating in response to fluid flowing through the outlet channel 264, thereby creating high frequency fluid pulses in the aspiration flow path 46. Thus, the oscillatory mode of the passive pressure oscillation assembly 244 may pulse fluid communication between the pressurized fluid source 16 and the aspiration flow path 46 at a second frequency concurrently with and different from the first frequency. In the illustrated embodiment, the fluid resonator 276 takes the form of a paddle wheel that spins in response to the flow of fluid through the outlet channel 264, although other types of fluid resonators are contemplated.

When the passive pressure oscillation assembly 244 is operating in the normal mode, the fluid-actuated valve 274 is configured for being in an open state in response to the lack of fluid flow through the aspiration shut-off channel 270, thereby allowing fluid communication between the aspiration source 14 and the aspiration flow path 46. In contrast, when the passive pressure oscillation assembly 244 is operating in the oscillatory mode, the fluid-actuated valve 274 is configured for being in a closed state in response to the presence of fluid flow through the aspiration shut-off channel 270, thereby preventing fluid communication between the aspiration source 14 and the aspiration flow path 46. In this manner, the high-frequency pulses generated by the fluid resonator 276 in the aspiration flow path 46 are not absorbed by the aspiration source 14, and instead fully propagate down the aspiration flow path 46 to the aspiration catheter 12. In the illustrated embodiment, the fluid-actuated valve 274 takes the form of a diaphragm valve, although other types of fluid actuated valves are contemplated.

The plunger assembly 258 interacts with the plunger cavity 254 to switch the passive pressure oscillation assembly 244 between the normal mode and the oscillatory mode in accordance with the timing diagram illustrated in **Fig. 14****.**

As illustrated in **Fig. 14****,** the aspiration source 14 is first activated, such that the aspiration catheter 12 is in a free-flow condition between arbitrary time t₀ and t₁, and the negative pressure differential between the absolute pressure in the aspiration flow path 46 and the external ambient pressure experienced by the aspiration catheter 12 (in this case, the negative pressure differential between the pressurized fluid source 16 and the aspiration flow path 46) is at a free-flow negative pressure differential where the aspiration catheter 12 is only ingesting blood. During this time, the passive pressure oscillation assembly 244 remains in the normal mode. In this manner, the aspiration efficiency of the system 10 is maximized during free-flow conditions.

Between arbitrary time t₁ and arbitrary time t₂, the thrombus 2 is actively being ingested into the distal end 30 of the aspiration catheter 12, such that the negative pressure differential between the absolute pressure in the aspiration flow path 46 and the external ambient pressure experienced by the aspiration catheter 12 drops below the free-flow negative pressure differential, but not below the negative activation pressure differential of the passive pressure oscillation assembly 244, which is designed for a no-flow or low-flow condition indicative of a clogged aspiration catheter 12 or flow anomaly in the aspiration conduit of the system 10. During arbitrary time t₀ and time t₂, the passive pressure oscillation assembly 44 remains in the normal mode.

The state of passive pressure oscillation assembly 244 illustrated in **Fig. 13A** during free-flow and ingestion (normal mode) corresponds to "State A" in the timing diagram of **Fig. 14****.** As illustrated in **Fig. 13A****,** in the absence of sufficient fluid pressure from the inlet channel 262 (via the inlet port 256a of the plunger cavity 254) on the first plunger head 280 of the plunger assembly 258, the spring 260 is configured for applying a biasing force to the plunger assembly 258 that maintains the first plunger head 280 and second plunger head 282 in closed positions within the plunger cavity 254.

In its closed position, the first plunger head 280 prevents fluid communication between the front plunger cavity region 284 and the center plunger cavity region 286 via the bypass channel 266, thereby preventing the flow of fluid from the pressurized fluid source 16, through the inlet channel 262, through the bypass channel 266, and into the center plunger cavity region 286. In its closed position, the first plunger head 280 also allows fluid communication between the aspiration flow path 46 and the center plunger cavity region 286 via the pressure equalization channel 272, thereby allowing the flow of fluid from the aspiration flow path 46 into the center plunger cavity region 286 to equalize the pressures of the aspiration flow path 46 and the center plunger cavity region 286. In this manner, the passive pressure oscillation assembly 244 may be reset at the beginning of each oscillation cycle.

In its closed position, the second plunger head 282 prevents fluid communication between the center plunger cavity region 286 and the aspiration flow path 46 via the outlet channel 264, thereby preventing the flow of fluid from the center plunger cavity region 286, through the outlet channel 264, and into the aspiration flow path 46. In its closed position, the second plunger head 282 also prevents fluid communication between the center plunger cavity region 286 and the fluid-actuated valve 274 via the aspiration shut-off channel 270, thereby preventing the flow of fluid from the plunger cavity 254 and through the aspiration shut-off channel 270, and allows fluid communication between the rear plunger cavity region 288 and the fluid-actuated valve 274 via the aspiration shut-off channel 270, thereby allowing the venting or backflow of fluid from the fluid-actuated valve 274. As such, the fluid-actuated valve 274 disposed in the aspiration flow path 46 will be switched to and maintained in its open state to, in turn, maintain fluid communication between the aspiration source 14 and the aspiration flow path 46.

Between arbitrary time t₁ and arbitrary time t₂, the thrombus 2 is actively being ingested into the distal end 30 of the aspiration catheter 12, such that the negative pressure differential between the absolute pressure in the aspiration flow path 46 and the external ambient pressure experienced by the aspiration catheter 12 drops below the free-flow negative pressure differential, but not below the negative activation pressure differential of the passive pressure oscillation assembly 244, which is designed for a no-flow or low-flow condition indicative of a clogged aspiration catheter 12 or flow anomaly in the aspiration conduit of the system 10. During arbitrary time t₀ and time t₂, the passive pressure oscillation assembly 44 remains in the normal mode, since the fluid pressure applied from the inlet channel 262 on the first plunger head 280 of the plunger assembly 258 is not sufficient enough to actively oppose the biasing force applied to the plunger assembly 258 by the spring 260 in a manner that displaces the first plunger head 280 and the second plunger head 282 from their closed positions.

At arbitrary time t₂, however, the aspiration catheter 12 becomes clogged with the thrombus 2, resulting in a precipitous decrease in the negative pressure differential between the absolute pressure in the aspiration flow path 46 and the external ambient pressure experienced by the aspiration catheter 12 to the negative activation pressure differential, which in the illustrated case, is at -75kPa. Thus, at or just after the arbitrary time t₂, the clogged aspiration catheter 12 (no-flow or low-flow condition) triggers the passive pressure oscillation assembly 244 to switch from the normal mode to the oscillatory mode, resulting in both low frequency pressure oscillations and high frequency pressure oscillations in the aspiration flow path 46 that cause pressure pulses to propagate down the aspiration conduit 24 of the aspiration catheter 12, thereby facilitating clearance of the clogged thrombus 2 at the distal end 30 of the aspiration catheter 12 at the arbitrary time t₄.

In particular, at arbitrary time t₃ₐ, the negative pressure differential between the absolute pressure in the aspiration flow path 46 and the external ambient pressure experienced by the aspiration catheter 12 reaches the negative activation pressure differential.

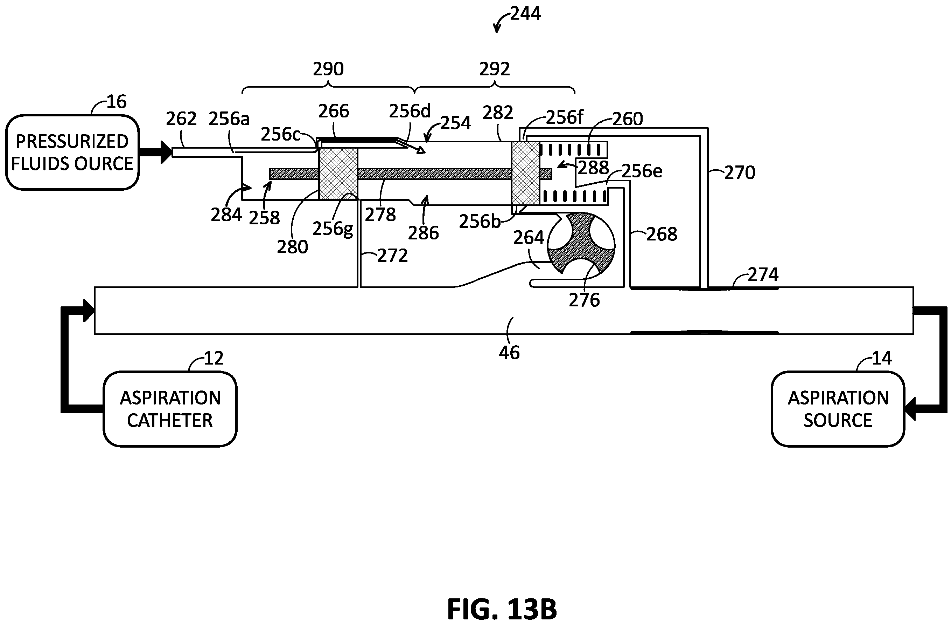

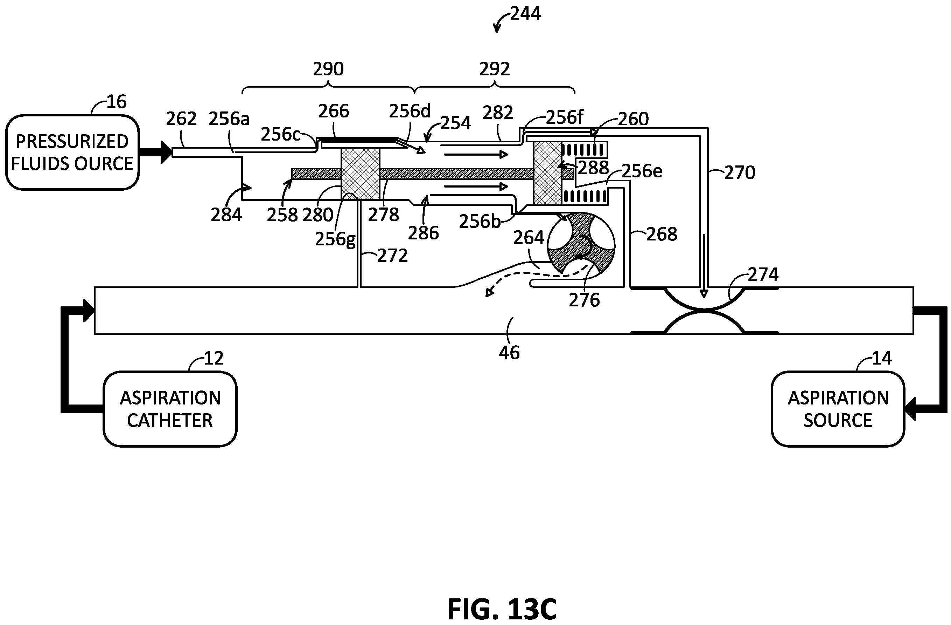

As illustrated in **Figs. 13B** and **13C****,** during the first stage of the operation of the passive pressure oscillation assembly 244 in the oscillatory mode, and in the presence of sufficient fluid pressure from the inlet channel 262 (via the inlet port 256a of the plunger cavity 254) on the first plunger head 280 of the plunger assembly 258, the biasing force applied by the spring 260 to the plunger assembly 258 is overcome to displace the first plunger head 280 and second plunger head 282 from closed positions to open positions within the plunger cavity 254.

In its open position, the first plunger head 280 allows fluid communication between the front plunger cavity region 284 and the center plunger cavity region 286 via the bypass channel 266, thereby allowing the flow of fluid from the pressurized fluid source 16, through the inlet channel 262, through the bypass channel 266, and into the center plunger cavity region 286. In its open position, the first plunger head 280 also prevents fluid communication between the aspiration flow path 46 and the center plunger cavity region 286 via the pressure equalization channel 272, thereby preventing a continuous flow of fluid into the aspiration flow path 46 from the center plunger cavity region 286 via the pressure equalization channel 272.

In its open position, the second plunger head 282 allows fluid communication between the center plunger cavity region 286 and the aspiration flow path 46 via the outlet channel 264, thereby allowing the flow of fluid from the center plunger cavity region 286, through the outlet channel 264, and into the aspiration flow path 46, and activating the fluid resonator 276. In its open position, the second plunger head 282 also allows fluid communication between the center plunger cavity region 286 and the fluid-actuated valve 274 via the aspiration shut-off channel 270, thereby allowing the flow of fluid from the plunger cavity 254 and through the aspiration shut-off channel 270. In its open position, the second plunger head 282 also preventing fluid communication between the rear plunger cavity region 288 and the fluid-actuated valve 274 via the aspiration shut-off channel 270, thereby preventing the venting or backflow of fluid from the aspiration shut-off valve 270 into the rear plunger cavity region 288. As such, the fluid-actuated valve 274 disposed in the aspiration flow path 46 will be switched to and maintained in its closed state to, in turn, preventing fluid communication between the aspiration source 14 and the aspiration flow path 46.

In this embodiment, the first plunger head 280 and the second plunger head 282 are displaced into their open positions in a two-step process.

In particular, as illustrated in **Fig. 13B****,** the fluid originating from the pressurized fluid source 16 applies pressure to the first plunger head 280, thereby displacing it from its closed position to its open position, and allowing fluid communication between the front plunger cavity region 284 and the center plunger cavity region 286 via the bypass channel 266. As a result, fluid flows from the pressurized fluid source 16, through the inlet channel 262, into the front plunger cavity region 284, through the bypass channel 266, and into the center plunger cavity region 286. The state of the passive pressure oscillation assembly 244 illustrated in **Fig. 13B** corresponds to "State B" in the timing diagram of **Fig. 14****.** The flow of fluid into the center plunger cavity region 286 applies additional force to the second plunger head 282, and thus, the spring 260, thereby displacing the second plunger head 282 further from its closed position to its fully open position (**Fig. 13C**), and allowing fluid communication between the center plunger cavity region 286 and the aspiration flow path 46 via the outlet channel 264, and the center plunger cavity region 286 and the fluid-actuated valve 274 via the aspiration shut-off channel 270. As a result, fluid flows from the center plunger cavity region 286, through the outlet channel 264, and into the aspiration flow path 46, and from the center plunger cavity region 286, and through the aspiration shut-off channel 270, to place the fluid actuated valve 274 in its closed state. The state of the passive pressure oscillation assembly 244 illustrated in **Fig. 13C** corresponds to "State C" in the timing diagram of **Fig. 14****.** It should be appreciated that, although the pressure between the front plunger cavity region 284 and the center plunger cavity region 286 becomes the same at State B (i.e., equalizes between State A and State B), because the surface area of the second plunger head 282 is greater than the surface area of the first plunger head 280, the force applied to the second plunger head 282 by the fluid in the center plunger cavity region 286 is greater than the force applied to the first plunger head 280 by the fluid in the front plunger cavity region 284. As a result, an additional net force is applied to the plunger assembly 258, and thus the spring 260, thereby "kicking" the second plunger head 282 from its closed position to its open position.

As can be seen from **Fig. 14****,** between arbitrary time t₃ₐ and arbitrary time t_{3b}, the flow of fluid into the aspiration flow path 46 causes a gradual increase in the negative pressure differential between the absolute pressure in the aspiration flow path 46 and the external ambient pressure experienced by the aspiration catheter 12 to the negative cessation pressure differential, which in the illustrated case, is at - 10kPa, which completes the first stage of the operation of the passive pressure oscillation assembly 244 in the oscillatory mode. Between arbitrary time t₃ₐ and arbitrary time t_{3b}, the negative pressure differential between the absolute pressure in the aspiration flow path 46 and the external ambient pressure experienced by the aspiration catheter 12 is also pulsed at a low amplitude, but high frequency, via operation of the fluid resonator 276.

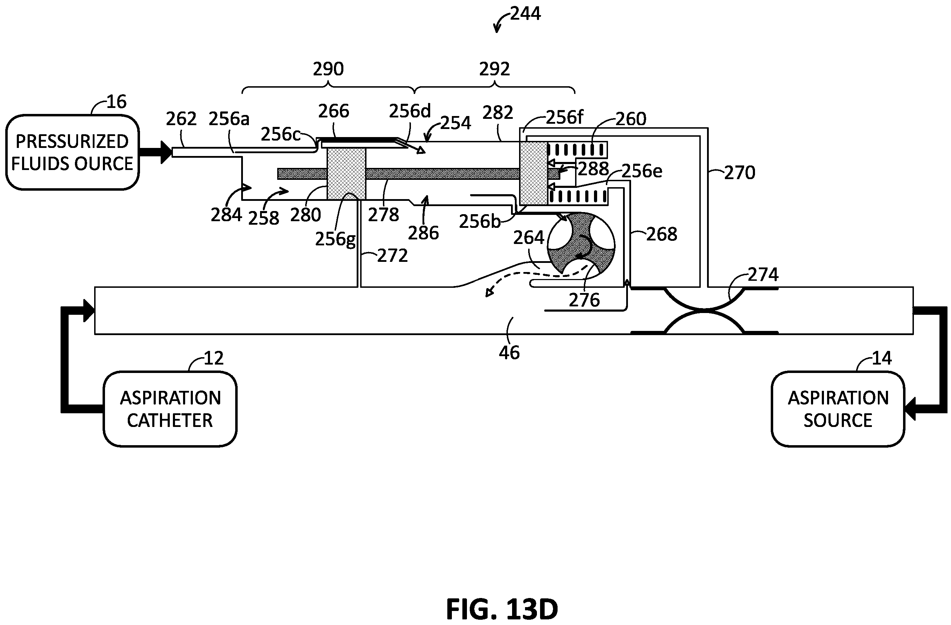

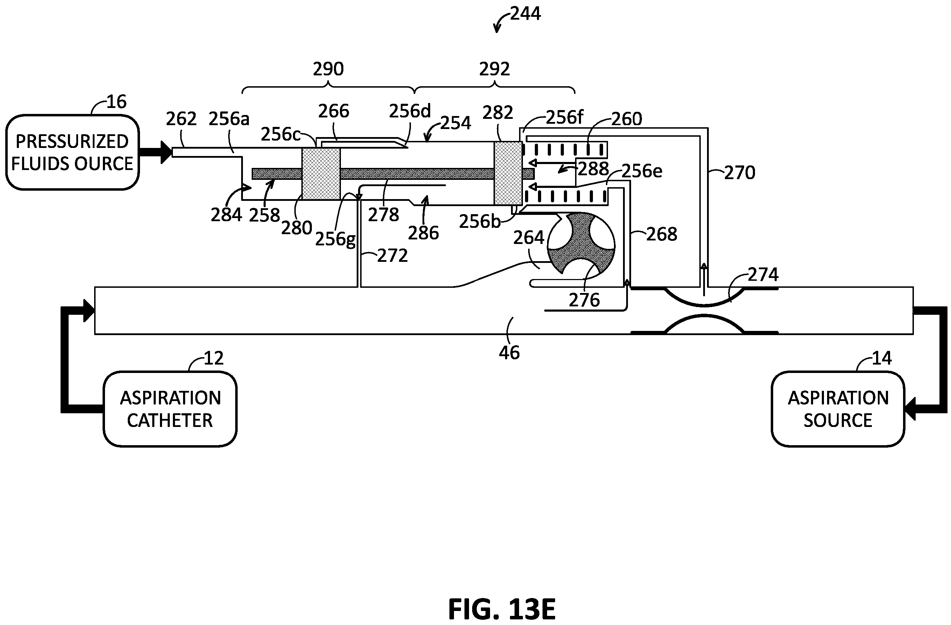

As illustrated in **Figs. 13D** and **13E****,** during the second stage of the operation of the passive pressure oscillation assembly 244 in the oscillatory mode, and in the presence of sufficient fluid pressure from the pressure tap channel 268 (via the pressure tap port 256e of the plunger cavity 254) on the second plunger head 282 of the plunger assembly 258, the biasing force applied by the spring 260 to the plunger assembly 258 is supplemented to displace the first plunger head 280 and second plunger head 282 from open positions back to closed positions within the plunger cavity 254. Notably, the pressure tap channel 268 is the main differential pressure tap (or sensing line), and is critical to the functioning of the passive pressure oscillation assembly 244. For any given negative pressure differential between the absolute pressure in the aspiration flow path 46 and the external ambient pressure experienced by the aspiration catheter 12, the net force on the plunger assembly 258 when the second plunger head 282 is in its open state, and thus the spring 260, is between the center plunger cavity region 286 and the rear plunger cavity region 288 due to the larger surface area of the second plunger head 282 relative to the surface area of the first plunger head 280. When the second plunger head 282 is in its closed state, the net force on the plunger assembly 258 is between the front plunger cavity region 284 and the rear plunger cavity region 288 and is less due to the smaller surface area of the first plunger head 280 relative to the surface area of the second plunger head 282.

In its closed position, the second plunger head 282 also prevents fluid communication between the center plunger cavity region 286 and the fluid-actuated valve 274 via the aspiration shut-off channel 270, thereby ceasing the flow of fluid from the plunger cavity 254 and through the aspiration shut-off channel 270 (**Fig. 13D**). As such, the fluid-actuated valve 274 disposed in the aspiration flow path 46 will be switched back in its closed state to, in turn, reinitiate fluid communication between the aspiration source 14 and the aspiration flow path 46. The state of the passive pressure oscillation assembly 244 illustrated in **Fig. 13D** corresponds to "State D" in the timing diagram of **Fig. 14****.** In its closed position, the second plunger head 282 also prevents fluid communication between the center plunger cavity region 286 and the aspiration flow path 46 via the outlet channel 264, thereby ceasing the flow of fluid from the center plunger cavity region 286, through the outlet channel 264, and into the aspiration flow path 46 (**Fig. 13E**). In its closed position, the second plunger head 282 also allows fluid communication between the rear plunger cavity region 288 and the aspiration shut-off channel 270, thereby allowing the venting or backflow of fluid from the fluid-actuated valve 274 into the rear plunger cavity region 288. As a result, the fluid-actuated valve 274 will be switched back in its closed state to, in turn, reinitiate fluid communication between the aspiration source 14 and the aspiration flow path 46.

In its closed position, the first plunger head 280 prevents fluid communication between the front plunger cavity region 284 and the center plunger cavity region 286 via the bypass channel 266, thereby ceasing the flow of fluid from the pressurized fluid source 16, through the inlet channel 262, through the bypass channel 266, and into the center plunger cavity region 286 (**Fig. 13E**). In its closed position, the first plunger head 280 also allows fluid communication between the aspiration flow path 46 and the center plunger cavity region 286 via the pressure equalization channel 272, thereby allowing the flow of fluid into the aspiration flow path 46 from the center plunger cavity region 286 to equalize the pressures of the aspiration flow path 46 and the center plunger cavity region 286 (**Fig. 13E**). The state of the passive pressure oscillation assembly 244 illustrated in **Fig. 13E** corresponds to "State E" in the timing diagram of **Fig. 14****.**

Notably, to ensure that the first plunger head 280 and the second plunger head 282 remain in open positions until the negative pressure differential between the absolute pressure in the aspiration flow path 46 and the external ambient pressure experienced by the aspiration catheter 12 reaches the negative cessation pressure differential at arbitrary time t_{3b}, the increased diameter of the second plunger head 282 relative to the diameter of the first plunger head 280 increases the pressure applied to the second plunger head 282 by the fluid in the pressure tap channel 268 required to displace the plunger assembly 258 in a manner that displaces the first plunger head 280 and the second plunger head 282 back into the closed positions.

Between arbitrary time t_{3b} and arbitrary time t_{3c}, the negative pressure differential between the absolute pressure in the aspiration flow path 46 and the external ambient pressure experienced by the aspiration catheter 12 precipitously decreases. If the thrombus 2 in the aspiration catheter 12 has not been cleared at time t_{3c}, the negative pressure differential between the absolute pressure in the aspiration flow path 46 and the external ambient pressure experienced by the aspiration catheter 12 continues to precipitously decrease to the negative activation pressure differential at arbitrary time t_{3d}, and the first and second stages of the operation of the passive pressure oscillation assembly 244 in the oscillatory mode is repeated. If the thrombus 2 in the aspiration catheter 12 has been cleared at time t_{3c}, the cleared aspiration catheter 12 (free-flow condition) triggers the passive pressure oscillation assembly 244 to switch from the oscillatory mode to the normal mode, ceasing pressure oscillations in the aspiration flow path 46, and thus ceasing pressure pulses from propagating down the aspiration conduit 24 of the aspiration catheter 12, shown at arbitrary time t₄.

Although particular embodiments have been shown and described herein, it will be understood by those skilled in the art that they are not intended to limit the disclosed inventions, and it will be obvious to those skilled in the art that various changes, permutations, and modifications may be made (e.g., the dimensions of various parts, combinations of parts) without departing from the scope of the disclosed inventions, which is to be defined only by the following claims. The specification and drawings are, accordingly, to be regarded in an illustrative rather than restrictive sense. The various embodiments shown and described herein are intended to cover alternatives, modifications, and equivalents of the disclosed inventions, which may be included within the scope of the appended claims.
aspiration flow path 46. In this manner, the high-frequency pulses generated by the fluid resonator 276 in the aspiration flow path 46 are not absorbed by the aspiration source 14, and instead fully propagate down the aspiration flow path 46 to the aspiration catheter 12. In the illustrated embodiment, the fluid-actuated valve 274 takes the form of a diaphragm valve, although other types of fluid actuated valves are contemplated.

The plunger assembly 258 interacts with the plunger cavity 254 to switch the passive pressure oscillation assembly 244 between the normal mode and the oscillatory mode in accordance with the timing diagram illustrated in **Fig. 14****.**

As illustrated in **Fig. 14****,** the aspiration source 14 is first activated, such that the aspiration catheter 12 is in a free-flow condition between arbitrary time t₀ and t₁, and the negative pressure differential between the absolute pressure in the aspiration flow path 46 and the external ambient pressure experienced by the aspiration catheter 12 (in this case, the negative pressure differential between the pressurized fluid source 16 and the aspiration flow path 46) is at a free-flow negative pressure differential where the aspiration catheter 12 is only ingesting blood. During this time, the passive pressure oscillation assembly 244 remains in the normal mode. In this manner, the aspiration efficiency of the system 10 is maximized during free-flow conditions.

Between arbitrary time t₁ and arbitrary time t₂, the thrombus 2 is actively being ingested into the distal end 30 of the aspiration catheter 12, such that the negative pressure differential between the absolute pressure in the aspiration flow path 46 and the external ambient pressure experienced by the aspiration catheter 12 drops below the free-flow negative pressure differential, but not below the negative activation pressure differential of the passive pressure oscillation assembly 244, which is designed for a no-flow or low-flow condition indicative of a clogged aspiration catheter 12 or flow anomaly in the aspiration conduit of the system 10. During arbitrary time t₀ and time t₂, the passive pressure oscillation assembly 44 remains in the normal mode.

The state of passive pressure oscillation assembly 244 illustrated in **Fig. 13A** during free-flow and ingestion (normal mode) corresponds to "State A" in the timing diagram of **Fig. 14****.** As illustrated in **Fig. 13A****,** in the absence of sufficient fluid pressure from the inlet channel 262 (via the inlet port 256a of the plunger cavity 254) on the first plunger head 280 of the plunger assembly 258, the spring 260 is configured for applying a biasing force to the plunger assembly 258 that maintains the first plunger head 280 and second plunger head 282 in closed positions within the plunger cavity 254.

In its closed position, the first plunger head 280 prevents fluid communication between the front plunger cavity region 284 and the center plunger cavity region 286 via the bypass channel 266, thereby preventing the flow of fluid from the pressurized fluid source 16, through the inlet channel 262, through the bypass channel 266, and into the center plunger cavity region 286. In its closed position, the first plunger head 280 also allows fluid communication between the flow aspiration path 46 and the center plunger cavity region 286 via the pressure equalization channel 272, thereby allowing the flow of fluid from the flow aspiration path 46 into the center plunger cavity region 286 to equalize the pressures of the flow aspiration path 46 and the center plunger cavity region 286. In this manner, the passive pressure oscillation assembly 244 may be reset at the beginning of each oscillation cycle.

In its closed position, the second plunger head 282 prevents fluid communication between the center plunger cavity region 286 and the aspiration flow path 46 via the outlet channel 264, thereby preventing the flow of fluid from the center plunger cavity region 286, through the outlet channel 264, and into the aspiration flow path 46. In its closed position, the second plunger head 282 also prevents fluid communication between the center plunger cavity region 286 and the fluid-actuated valve 274 via the aspiration shut-off channel 270, thereby preventing the flow of fluid from the plunger cavity 254 and through the aspiration shut-off channel 270, and allows fluid communication between the rear plunger cavity region 288 and the fluid-actuated valve 274 via the aspiration shut-off channel 270, thereby allowing the venting or backflow of fluid from the fluid-actuated valve 274. As such, the fluid-actuated valve 274 disposed in the aspiration flow path 46 will be switched to and maintained in its open state to, in turn, maintain fluid communication between the aspiration source 14 and the aspiration flow path 46.

Between arbitrary time t₁ and arbitrary time t₂, the thrombus 2 is actively being ingested into the distal end 30 of the aspiration catheter 12, such that the negative pressure differential between the absolute pressure in the aspiration flow path 46 and the external ambient pressure experienced by the aspiration catheter 12 drops below the free-flow negative pressure differential, but not below the negative activation pressure differential of the passive pressure oscillation assembly 244, which is designed for a no-flow or low-flow condition indicative of a clogged aspiration catheter 12 or flow anomaly in the aspiration conduit of the system 10. During arbitrary time t₀ and time t₂, the passive pressure oscillation assembly 44 remains in the normal mode, since the fluid pressure applied from the inlet channel 262 on the first plunger head 280 of the plunger assembly 258 is not sufficient enough to actively oppose the biasing force applied to the plunger assembly 258 by the spring 260 in a manner that displaces the first plunger head 280 and the second plunger head 282 from their closed positions.

At arbitrary time t₂, however, the aspiration catheter 12 becomes clogged with the thrombus 2, resulting in a precipitous decrease in the negative pressure differential between the absolute pressure in the aspiration flow path 46 and the external ambient pressure experienced by the aspiration catheter 12 to the negative activation pressure differential, which in the illustrated case, is at -75kPa. Thus, at or just after the arbitrary time t₂, the clogged aspiration catheter 12 (no-flow or low-flow condition) triggers the passive pressure oscillation assembly 244 to switch from the normal mode to the oscillatory mode, resulting in both low frequency pressure oscillations and high frequency pressure oscillations in the aspiration flow path 46 that cause pressure pulses to propagate down the aspiration conduit 24 of the aspiration catheter 12, thereby facilitating clearance of the clogged thrombus 2 at the distal end 24 of the aspiration catheter 12 at the arbitrary time t₄.

In particular, at arbitrary time t₃ₐ, the negative pressure differential between the absolute pressure in the aspiration flow path 46 and the external ambient pressure experienced by the aspiration catheter 12 reaches the negative activation pressure differential.

As illustrated in **Figs. 13B** and **13C****,** during the first stage of the operation of the passive pressure oscillation assembly 244 in the oscillatory mode, and in the presence of sufficient fluid pressure from the inlet channel 262 (via the inlet port 256a of the plunger cavity 254) on the first plunger head 280 of the plunger assembly 258, the biasing force applied by the spring 260 to the plunger assembly 258 is overcome to displace the first plunger head 280 and second plunger head 282 from closed positions to open positions within the plunger cavity 254.

In its open position, the first plunger head 280 allows fluid communication between the front plunger cavity region 284 and the center plunger cavity region 286 via the bypass channel 266, thereby allowing the flow of fluid from the pressurized fluid source 16, through the inlet channel 262, through the bypass channel 266, and into the center plunger cavity region 286. In its open position, the first plunger head 280 also prevents fluid communication between the flow aspiration path 46 and the center plunger cavity region 286 via the pressure equalization channel 272, thereby preventing a continuous flow of fluid into the flow aspiration path 46 from the center plunger cavity region 286 via the pressure equalization channel 272.

In its open position, the second plunger head 282 allows fluid communication between the center plunger cavity region 286 and the aspiration flow path 46 via the outlet channel 264, thereby allowing the flow of fluid from the center plunger cavity region 286, through the outlet channel 264, and into the aspiration flow path 46, and activating the fluid resonator 276. In its open position, the second plunger head 282 also allows fluid communication between the center plunger cavity region 286 and the fluid-actuated valve 274 via the aspiration shut-off channel 270, thereby allowing the flow of fluid from the plunger cavity 254 and through the aspiration shut-off channel 270. In its open position, the second plunger head 282 also preventing fluid communication between the rear plunger cavity region 288 and the fluid-actuated valve 274 via the aspiration shut-off channel 270, thereby preventing the venting or backflow of fluid from the aspiration shut-off valve 270 into the rear plunger cavity region 288. As such, the fluid-actuated valve 274 disposed in the aspiration flow path 46 will be switched to and maintained in its closed state to, in turn, preventing fluid communication between the aspiration source 14 and the aspiration flow path 46.

In this embodiment, the first plunger head 280 and the second plunger head 280 are displaced into their open positions in a two-step process.

In particular, as illustrated in **Fig. 13B****,** the fluid originating from the pressurized fluid source 16 applies pressure to the first plunger head 280, thereby displacing it from its closed position to its open position, and allowing fluid communication between the front plunger cavity region 284 and the center plunger cavity region 286 via the bypass channel 266. As a result, fluid flows from the pressurized fluid source 16, through the inlet channel 262, into the front plunger cavity region 284, through the bypass channel 266, and into the center plunger cavity region 286. The state of the passive pressure oscillation assembly 244 illustrated in **Fig. 13B** corresponds to "State B" in the timing diagram of **Fig. 14****.** The flow of fluid into the center plunger cavity region 286 applies additional force to the second plunger head 280, and thus, the spring 260, thereby displacing the second plunger head 280 further from its closed position to its fully open position (**Fig. 13C**), and allowing fluid communication between the center plunger cavity region 286 and the aspiration flow path 46 via the outlet channel 264, and the center plunger cavity region 286 and the fluid-actuated valve 274 via the aspiration shut-off channel 270. As a result, fluid flows from the center plunger cavity region 286, through the outlet channel 264, and into the aspiration flow path 46, and from the center plunger cavity region 286, and through the aspiration shut-off channel 270, to place the fluid actuated valve 274 in its closed state. The state of the passive pressure oscillation assembly 244 illustrated in **Fig. 13C** corresponds to "State C" in the timing diagram of **Fig. 14****.** It should be appreciated that, although the pressure between the front plunger cavity region 284 and the center plunger cavity region 286 becomes the same at State B (i.e., equalizes between State A and State B), because the surface area of the second plunger head 280 is greater than the surface area of the first plunger head 280, the force applied to the second plunger head 280 by the fluid in the center plunger cavity region 286 is greater than the force applied to the first plunger head 280 by the fluid in the front plunger cavity region 284. As a result, an additional net force is applied to the plunger assembly 258, and thus the spring 260, thereby "kicking" the second plunger head 280 from its closed position to its open position.

As can be seen from **Fig. 14****,** between arbitrary time t₃ₐ and arbitrary time t_{3b}, the flow of fluid into the aspiration flow path 46 causes a gradual increase in the negative pressure differential between the absolute pressure in the aspiration flow path 46 and the external ambient pressure experienced by the aspiration catheter 12 to the negative cessation pressure differential, which in the illustrated case, is at -10kPa, which completes the first stage of the operation of the passive pressure oscillation assembly 244 in the oscillatory mode. Between arbitrary time t₃ₐ and arbitrary time t_{3b}, the negative pressure differential between the absolute pressure in the aspiration flow path 46 and the external ambient pressure experienced by the aspiration catheter 12 is also pulsed at a low amplitude, but high frequency, via operation of the fluid resonator 276.

As illustrated in **Figs. 13D** and **13E****,** during the second stage of the operation of the passive pressure oscillation assembly 244 in the oscillatory mode, and in the presence of sufficient fluid pressure from the pressure tap channel 268 (via the pressure tap port 256e of the plunger cavity 254) on the second plunger head 282 of the plunger assembly 258, the biasing force applied by the spring 260 to the plunger assembly 258 is supplemented to displace the first plunger head 280 and second plunger head 282 from open positions back to closed positions within the plunger cavity 254. Notably, the pressure tap channel 268 is the main differential pressure tap (or sensing line), and is critical to the functioning of the passive pressure oscillation assembly 244. For any given negative pressure differential between the absolute pressure in the aspiration flow path 46 and the external ambient pressure experienced by the aspiration catheter 12, the net force on the plunger assembly 258 when the second plunger head 282 is in its open state, and thus the spring 260, is between the center plunger cavity region 286 and the rear plunger cavity region 288 due to the larger surface area of the second plunger head 282 relative to the surface area of the first plunger head 280. When the second plunger head 282 is in its closed state, the net force on the plunger assembly 258 is between the front plunger cavity region 284 and the rear plunger cavity region 288 and is less due to the smaller surface area of the first plunger head 280 relative to the surface area of the second plunger head 280.

In its closed position, the second plunger head 282 also prevents fluid communication between the center plunger cavity region 286 and the fluid-actuated valve 274 via the aspiration shut-off channel 270, thereby ceasing the flow of fluid from the plunger cavity 254 and through the aspiration shut-off channel 270 (**Fig. 13D**). As such, the fluid-actuated valve 274 disposed in the aspiration flow path 46 will be switched back in its closed state to, in turn, reinitiate fluid communication between the aspiration source 14 and the aspiration flow path 46. The state of the passive pressure oscillation assembly 244 illustrated in **Fig. 13D** corresponds to "State D" in the timing diagram of **Fig. 14****.** In its closed position, the second plunger head 282 also prevents fluid communication between the center plunger cavity region 286 and the aspiration flow path 46 via the outlet channel 264, thereby ceasing the flow of fluid from the center plunger cavity region 286, through the outlet channel 264, and into the aspiration flow path 46 (**Fig. 13E**). In its closed position, the second plunger head 282 also allows fluid communication between the rear plunger cavity region 288 and the aspiration shut-off channel 270, thereby allowing the venting or backflow of fluid from the fluid-actuated valve 274 into the rear plunger cavity region 288. As a result, the fluid-actuated valve 274 will be switched back in its closed state to, in turn, reinitiate fluid communication between the aspiration source 14 and the aspiration flow path 46.

In its closed position, the first plunger head 280 prevents fluid communication between the front plunger cavity region 284 and the center plunger cavity region 286 via the bypass channel 266, thereby ceasing the flow of fluid from the pressurized fluid source 16, through the inlet channel 262, through the bypass channel 266, and into the center plunger cavity region 286 (**Fig. 13E**). In its closed position, the first plunger head 280 also allows fluid communication between the flow aspiration path 46 and the center plunger cavity region 286 via the pressure equalization channel 272, thereby allowing the flow of fluid into the flow aspiration path 46 from the center plunger cavity region 286 to equalize the pressures of the flow aspiration path 46 and the center plunger cavity region 286 (**Fig. 13E**). The state of the passive pressure oscillation assembly 244 illustrated in **Fig. 13E** corresponds to "State E" in the timing diagram of **Fig. 14****.**

Notably, to ensure that the first plunger head 280 and the second plunger head 282 remain in open positions until the negative pressure differential between the absolute pressure in the aspiration flow path 46 and the external ambient pressure experienced by the aspiration catheter 12 reaches the negative cessation pressure differential at arbitrary time t_{3b}, the increased diameter of the second plunger head 282 relative to the diameter of the first plunger head 280 increases the pressure applied to the second plunger head 282 by the fluid in the pressure tap channel 268 required to displace the plunger assembly 258 in a manner that displaces the first plunger head 280 and the second plunger head 280 back into the closed positions.

Between arbitrary time t_{3b} and arbitrary time t_{3c}, the negative pressure differential between the absolute pressure in the aspiration flow path 46 and the external ambient pressure experienced by the aspiration catheter 12 precipitously decreases. If the thrombus 2 in the aspiration catheter 12 has not been cleared at time t_{3c}, the negative pressure differential between the absolute pressure in the aspiration flow path 46 and the external ambient pressure experienced by the aspiration catheter 12 continues to precipitously decrease to the negative activation pressure differential at arbitrary time t_{3d}, and the first and second stages of the operation of the passive pressure oscillation assembly 244 in the oscillatory mode is repeated. If the thrombus 2 in the aspiration catheter 12 has been cleared at time t_{3c}, the cleared aspiration catheter 12 (free-flow condition) triggers the passive pressure oscillation assembly 244 to switch from the oscillatory mode to the normal mode, ceasing pressure oscillations in the aspiration flow path 46, and thus ceasing pressure pulses from propagating down the aspiration conduit 24 of the aspiration catheter 12, shown at arbitrary time t₄.

Although particular embodiments have been shown and described herein, it will be understood by those skilled in the art that they are not intended to limit the disclosed inventions, and it will be obvious to those skilled in the art that various changes, permutations, and modifications may be made (e.g., the dimensions of various parts, combinations of parts) without departing from the scope of the disclosed inventions, which is to be defined only by the following claims. The specification and drawings are, accordingly, to be regarded in an illustrative rather than restrictive sense. The various embodiments shown and described herein are intended to cover alternatives, modifications, and equivalents of the disclosed inventions, which are included within the scope of the appended claims.

## Claims

1. A manifold (20) comprising:
an aspiration outlet (38) configured for being fluidly coupled an aspiration source (14);
an aspiration inlet (36) configured for being fluidly coupled to an aspiration catheter (12), such that an aspiration flow path (46) is formed between the aspiration catheter (12) and the aspiration source (14), wherein the aspiration flow path (46) has a free flow absolute pressure that creates a negative free flow pressure differential between the aspiration outlet (38) and the aspiration inlet (36);
a relief inlet (40) configured for being fluidly coupled to a pressurized fluid source (16); and
a pressure oscillation assembly (244) fluidly coupled between the relief inlet (40) and the aspiration flow path (46),
**characterized in that**
the pressure oscillation assembly (244) is configured for being operated between a normal mode that prevents fluid communication between the pressurized fluid source (16) and the aspiration flow path (46), and an oscillatory mode that pulses fluid communication between the pressurized fluid source (16) and the aspiration flow path (46), such that a negative pressure differential between the aspiration outlet (38) and the aspiration inlet (36) oscillates between a first negative pressure differential less than the free flow pressure differential and a second negative pressure differential greater than the free flow pressure differential when the pressure oscillation assembly (244) is operated in the oscillatory mode.

2. The manifold (20) of claim 1, wherein the first negative pressure differential is an activation pressure differential that triggers the pressure oscillation assembly (244) to gradually increase the negative pressure differential between the aspiration outlet (38) and the aspiration inlet (36), and the second negative pressure differential is a cessation pressure differential that triggers the pressure oscillation assembly (244) to gradually decrease the negative pressure differential between the aspiration outlet (38) and the aspiration inlet (36).

3. The manifold (20) of one of claims 1 and 2, wherein the second pressure differential is 40kPa-90kPa greater than the first pressure differential.

4. The manifold (20) of any of claims 1-3, wherein the pressure oscillation assembly (244) is configured for pulsing the fluid communication between the pressurized fluid source (16) and the aspiration flow path (46) concurrently at a first frequency and at a second frequency different from the first frequency.

5. The manifold (20) of claim 4, wherein the first frequency is in the range of 0.2Hz-10Hz, and the second frequency is in the range of 100Hz-400Hz.

6. The manifold (20) of one of claims 1-5, wherein the pressure oscillation assembly (244) is a passive pressure oscillation assembly configured for being triggered to automatically switch from the normal mode to the oscillatory mode in response to a clogged thrombus (2) in the aspiration catheter (12).

7. The manifold (20) of claim 6, wherein the passive pressure oscillation assembly (244) is configured for being triggered to automatically switch from the oscillatory mode to the normal mode in response to a removal of the clogged thrombus (2) from the aspiration catheter (12).

8. The manifold (20) of either of claim 6 or 7, wherein the passive pressure oscillation assembly (244) comprises:
a plunger cavity (254) in fluid communication between the relief inlet (40) and the aspiration flow path (46); and
a plunger assembly (258) slidably disposed within the plunger cavity (254), the plunger assembly (258) configured for, when the passive pressure oscillation assembly (244) is in the normal mode, preventing fluid communication through the plunger cavity (254) between the pressurized fluid source (16) and the aspiration flow path (46), and when the passive pressure oscillation assembly (244) is in the oscillatory mode, pulsing fluid communication between the pressurized fluid source (16) and the aspiration flow path (46) at a first frequency.

9. The manifold of claim 8, wherein the plunger assembly (258) includes a rod (278), a first plunger head (280) affixed to the rod (278), and a second plunger head (282) affixed to the rod (278) in a spaced apart relationship with the first plunger head 8280), thereby forming a front plunger cavity (254) region, a center plunger cavity (254) region between the first plunger head (280) and the second plunger head (282), and a rear plunger cavity region (288) within the plunger cavity (254), the passive pressure oscillation assembly (244) further comprising:
an inlet channel (262) in fluid communication between the relief inlet (40) and the front plunger cavity region (284);
an outlet channel (264) conditionally in fluid communication between the center plunger cavity region (286) and the aspiration flow path 846);
a bypass channel (266) conditionally in fluid communication between the front plunger cavity region (284) and the center plunger cavity region (286);
wherein the plunger assembly (258) is configured for, when the passive pressure oscillation assembly (244) is in the normal mode, preventing fluid communication through the plunger cavity (254) between the pressurized fluid source (16) and the aspiration flow path (46) by maintaining the first plunger head (280) in a closed position within the plunger cavity (254), thereby preventing fluid communication between the front plunger cavity region (284) and the center plunger cavity region (286) via the bypass channel (266), and maintaining the second plunger head (282) in a closed position within the plunger cavity (254), thereby preventing fluid communication between the center plunger cavity region (286) and the aspiration flow path (46) via the outlet channel (264);
wherein the plunger assembly (258) is configured for, when the passive pressure oscillation assembly (244) is in the oscillatory mode, pulsing fluid communication between the pressurized fluid source (16) and the aspiration flow path (46) at a first frequency by displacing the first plunger head (280) from the closed position to an open position within the plunger cavity (254), thereby allowing fluid communication between the front plunger cavity region (284) and the center plunger cavity region (286) via the bypass channel (266), and further displacing the second plunger head (282) from the closed position to an open position within the plunger cavity (254), thereby allowing fluid communication between the center plunger cavity region (286) and the aspiration flow path (46), and then displacing the first plunger head (280) and the second plunger head (282) from the open positions back to the closed positions.

10. The manifold (20) of claim 9, wherein the passive pressure oscillation assembly (244) further comprises a spring (260) configured for applying a biasing force to the plunger assembly (258) during operation of the passive pressure oscillation assembly (244) in the normal mode that maintains the first plunger head 8280) and the second plunger head (282) in the closed positions within the plunger cavity (254), wherein the plunger assembly (258), during operation of the passive pressure oscillation assembly (244) in the oscillatory mode, is configured for, in response to pressure applied by fluid originating from the pressurized fluid source (16) to the plunger assembly (258) via the inlet channel (262), overcoming the biasing force applied by the spring (260) to the plunger assembly (258) to displace the first plunger head (280) and second plunger head (282) from the closed positions to the open positions within the plunger cavity (254), and then supplementing the biasing force applied by the spring (260) to the plunger assembly (258) to displace the first plunger head (280) and the second plunger head (282) from the open positions back to the closed positions.

11. The manifold (20) of claim 10, wherein the passive pressure oscillation assembly (244) further comprises a pressure tap channel (268) in fluid communication between the aspiration flow path (46) and the rear plunger cavity region (288), wherein the plunger assembly (258), during operation of the passive pressure oscillation assembly (244) in the oscillatory mode, is configured for, in response to pressure applied by fluid originating from the aspiration flow path (46) to the plunger assembly (258) via the pressure tap channel (268), supplementing the biasing force applied by the spring (260) to the plunger assembly (258) to displace the first plunger head (280) and the second plunger head (282) from the open positions back to the closed positions.

12. The manifold (20) of any of claims 9-11, wherein the plunger cavity (254) has a first portion (290) having a diameter, and a second portion having a diameter greater than the diameter of the first portion (290), the first plunger head (280) has a diameter, and the second plunger head (282) has a diameter greater than the diameter of the first plunger head (280), wherein the first plunger head (280) is configured for being displaced within the first portion (290), and the second plunger head (282) is configured for being displaced within the second portion (292).

13. The manifold (20) of any of claims 9-12, further comprising:
a fluid-actuated valve (274) disposed within the aspiration flow path (46); and
an aspiration shutoff channel (270) conditionally in fluid communication between the center plunger cavity region (286) and the fluid-actuated valve (274);
wherein the second plunger head (282), when in the closed position, is configured for preventing fluid communication between the center plunger cavity region (286) and the fluid-actuated valve (274) via the aspiration shutoff channel (270), and allowing fluid communication between the rear plunger cavity region (288) and the fluid-actuated valve (274) via the aspiration shutoff channel (270), and when in the open position, is configured for allowing fluid communication between the center plunger cavity region (286) and the fluid-actuated valve (274) via the aspiration shutoff channel (270), and preventing fluid communication between the rear plunger cavity region (288) and the fluid-actuated valve (274) via the aspiration shutoff channel (270).

14. The manifold (20) of any of claims 9-13, further comprising a pressure equalization channel (272) conditionally in fluid communication between the aspiration flow path (46) and the center plunger cavity region (286), wherein the first plunger head (280), when in the closed position, is configured for allowing fluid communication between the aspiration flow path (46) and the center plunger cavity region (286), and when in the open position, is configured for preventing fluid communication between the aspiration flow path (46) and the center plunger cavity region (286).

15. The manifold (20) of any of claims 9-14, further comprising a resonator (276) disposed in the outlet channel (264), wherein the resonator, when the passive pressure oscillation assembly (244) is in the oscillatory mode, is configured for pulsing fluid communication between the plunger cavity (254) and the aspiration flow path (46) at a second frequency different from the first frequency.

## Patentansprüche

1. Ein Verteiler (20), umfassend:
einen Absauganschluss (38), konfiguriert zur fluidischen Kopplung an eine Absaugquelle (14);
einen Absaugeinlass (36), konfiguriert zur fluidischen Kopplung an einen Absaugkatheter (12), so dass ein Absaugströmungsweg (46) zwischen dem Absaugkatheter (12) und der Absaugquelle (14) gebildet wird, wobei der Absaugströmungsweg (46) einen freien Durchflussabsolutdruck hat, der eine negative freie Durchflussdruckdifferenz zwischen dem Absauganschluss (38) und dem Absaugeinlass (36) erzeugt;
einen Entlastungseinlass (40), konfiguriert zur fluidischen Kopplung an eine Druckflüssigkeitsquelle (16); und
eine Druckoszillationsanordnung (244), fluidisch gekoppelt zwischen dem Entlastungseinlass (40) und dem Absaugströmungsweg (46),
**gekennzeichnet durch**, dass
die Druckoszillationsanordnung (244) konfiguriert ist, um zwischen einem Normalmodus, der die Fluidkommunikation zwischen der Druckflüssigkeitsquelle (16) und dem Absaugströmungsweg (46) verhindert, und einem Oszillationsmodus, der die Fluidkommunikation zwischen der Druckflüssigkeitsquelle (16) und dem Absaugströmungsweg (46) pulsiert, so betrieben zu werden, dass eine negative Druckdifferenz zwischen dem Absauganschluss (38) und dem Absaugeinlass (36) zwischen einer ersten negativen Druckdifferenz, kleiner als die freie Durchflussdruckdifferenz, und einer zweiten negativen Druckdifferenz, größer als die freie Durchflussdruckdifferenz, oszilliert, wenn die Druckoszillationsanordnung (244) im Oszillationsmodus betrieben wird.

2. Der Verteiler (20) nach Anspruch 1, wobei die erste negative Druckdifferenz eine Aktivierungsdruckdifferenz ist, die die Druckoszillationsanordnung (244) veranlasst, die negative Druckdifferenz zwischen dem Absauganschluss (38) und dem Absaugeinlass (36) allmählich zu erhöhen, und die zweite negative Druckdifferenz eine Beendigungsdruckdifferenz ist, die die Druckoszillationsanordnung (244) veranlasst, die negative Druckdifferenz zwischen dem Absauganschluss (38) und dem Absaugeinlass (36) allmählich zu verringern.

3. Der Verteiler (20) nach einem der Ansprüche 1 oder 2, wobei die zweite Druckdifferenz 40kPa-90kPa größer als die erste Druckdifferenz ist.

4. Der Verteiler (20) nach einem der Ansprüche 1-3, wobei die Druckoszillationsanordnung (244) konfiguriert ist, um die Fluidkommunikation zwischen der Druckflüssigkeitsquelle (16) und dem Absaugströmungsweg (46) gleichzeitig mit einer ersten Frequenz und einer zweiten Frequenz, die von der ersten Frequenz verschieden ist, zu pulsen.

5. Der Verteiler (20) nach Anspruch 4, wobei die erste Frequenz im Bereich von 0,2Hz-10Hz liegt und die zweite Frequenz im Bereich von 100Hz-400Hz liegt.

6. Der Verteiler (20) nach einem der Ansprüche 1-5, wobei die passive Druckoszillationsanordnung (244) so konfiguriert ist, dass sie ausgelöst wird, um automatisch vom Normalmodus in den Oszillationsmodus zu wechseln, sofern ein verstopfter Thrombus (2) im Absaugkatheter (12) vorliegt.

7. Der Verteiler (20) nach Anspruch 6, wobei die passive Druckoszillationsanordnung (244) so konfiguriert ist, dass sie ausgelöst wird, um automatisch vom Oszillationsmodus in den Normalmodus zu wechseln, wenn die Verstopfung des Thrombus (2) im Absaugkatheter (12) entfernt wird.

8. Der Verteiler (20) nach einem der Ansprüche 6 oder 7, wobei die passive Druckoszillationsanordnung (244) Folgendes umfasst:
einen Kolbenraum (254), der fluidisch zwischen dem Entlastungseinlass (40) und dem Absaugströmungsweg (46) kommuniziert; und
eine Kolbenanordnung (258), die verschiebbar innerhalb des Kolbenraums (254) angeordnet ist, wobei die Kolbenanordnung (258) so konfiguriert ist, dass sie, wenn die passive Druckoszillationsanordnung (244) im Normalmodus ist, die Fluidkommunikation durch den Kolbenraum (254) zwischen der Druckflüssigkeitsquelle (16) und dem Absaugströmungsweg (46) verhindert und, wenn die passive Druckoszillationsanordnung (244) im Oszillationsmodus ist, die Fluidkommunikation zwischen der Druckflüssigkeitsquelle (16) und dem Absaugströmungsweg (46) mit einer ersten Frequenz pulsiert.

9. Der Verteiler (20) nach Anspruch 8, wobei die Kolbenanordnung (258) eine Stange (278), einen ersten Kolbenkopf (280), der an der Stange (278) befestigt ist, und einen zweiten Kolbenkopf (282), der in einem Abstand zum ersten Kolbenkopf (280) an der Stange (278) befestigt ist, um einen vorderen Kolbenraumabschnitt (284), einen zentralen Kolbenraumabschnitt (286) zwischen dem ersten Kolbenkopf (280) und dem zweiten Kolbenkopf (282) und einen hinteren Kolbenraumabschnitt (288) innerhalb des Kolbenraums (254) zu bilden, wobei die passive Druckoszillationsanordnung (244) ferner umfasst:
einen Einlasskanal (262), der fluidisch zwischen dem Entlastungseinlass (40) und dem vorderen Kolbenraumabschnitt (284) kommuniziert;
einen Auslasskanal (264), der bedingt fluidisch zwischen dem zentralen Kolbenraumabschnitt (286) und dem Absaugströmungsweg (46) kommuniziert;
einen Bypass-Kanal (266), der bedingt fluidisch zwischen dem vorderen Kolbenraumabschnitt (284) und dem zentralen Kolbenraumabschnitt (286) kommuniziert;
wobei die Kolbenanordnung (258) dafür konfiguriert ist, um, wenn die passive Druckoszillationsanordnung (244) im Normalmodus ist, die fluidische Kommunikation durch den Kolbenraum (254) zwischen der Druckflüssigkeitsquelle (16) und dem Absaugströmungsweg (46) zu verhindern, indem der erste Kolbenkopf (280) in einer geschlossenen Position innerhalb des Kolbenraums (254) gehalten wird, wodurch die fluidische Kommunikation zwischen dem vorderen Kolbenraumabschnitt (284) und dem zentralen Kolbenraumabschnitt (286) über den Bypass-Kanal (266) verhindert wird, und der zweite Kolbenkopf (282) in einer geschlossenen Position innerhalb des Kolbenraums (254) gehalten wird, wodurch die fluidische Kommunikation zwischen dem zentralen Kolbenraumabschnitt (286) und dem Absaugströmungsweg (46) über den Auslasskanal (264) verhindert wird;
wobei die Kolbenanordnung (258) dafür konfiguriert ist, um, wenn die passive Druckoszillationsanordnung (244) im Oszillationsmodus ist, die fluidische Kommunikation zwischen der Druckflüssigkeitsquelle (16) und dem Absaugströmungsweg (46) mit einer ersten Frequenz zu pulsen, indem der erste Kolbenkopf (280) von der geschlossenen Position in eine offene Position innerhalb des Kolbenraums (254) verschoben wird, wodurch die fluidische Kommunikation zwischen dem vorderen Kolbenraumabschnitt (284) und dem zentralen Kolbenraumabschnitt (286) über den Bypass-Kanal (266) ermöglicht wird, und der zweite Kolbenkopf (282) weiter von der geschlossenen Position in eine offene Position innerhalb des Kolbenraums (254) verschoben wird, wodurch die fluidische Kommunikation zwischen dem zentralen Kolbenraumabschnitt (286) und dem Absaugströmungsweg (46) ermöglicht wird, und dann der erste Kolbenkopf (280) und der zweite Kolbenkopf (282) von den offenen Positionen zurück in die geschlossenen Positionen verschoben wird.

10. Der Verteiler (20) nach Anspruch 9, wobei die passive Druckoszillationsanordnung (244) ferner eine Feder (260) umfasst, die dafür konfiguriert ist, eine Vorspannkraft auf die Kolbenanordnung (258) während des Betriebs der passiven Druckoszillationsanordnung (244) im Normalmodus auszuüben, wodurch der erste Kolbenkopf (280) und der zweite Kolbenkopf (282) in den geschlossenen Positionen innerhalb des Kolbenraums (254) gehalten werden, wobei die Kolbenanordnung (258), während des Betriebs der passiven Druckoszillationsanordnung (244) im Oszillationsmodus, dafür konfiguriert ist, in Reaktion auf den durch Fluid aus der Druckflüssigkeitsquelle (16) auf die Kolbenanordnung (258) über den Einlasskanal (262) ausgeübten Druck, die Vorspannkraft der Feder (260) zu überwinden, um den ersten Kolbenkopf (280) und den zweiten Kolbenkopf (282) von den geschlossenen Positionen in die offenen Positionen innerhalb des Kolbenraums (254) zu verschieben, und dann die durch die Feder (260) auf die Kolbenanordnung (258) ausgeübte Vorspannkraft zu unterstützen, um den ersten Kolbenkopf (280) und den zweiten Kolbenkopf (282) von den offenen Positionen zurück in die geschlossenen Positionen zu verschieben.

11. Der Verteiler (20) nach Anspruch 10, wobei die passive Druckoszillationsanordnung (244) ferner einen Druckabgriff-Kanal (268) umfasst, der fluidisch zwischen dem Absaugströmungsweg (46) und dem hinteren Kolbenraumabschnitt (288) kommuniziert, wobei die Kolbenanordnung (258), während des Betriebs der passiven Druckoszillationsanordnung (244) im Oszillationsmodus, dafür konfiguriert ist, in Reaktion auf den durch Fluid aus dem Absaugströmungsweg (46) auf die Kolbenanordnung (258) über den Druckabgriff-Kanal (268) ausgeübten Druck, die durch die Feder (260) auf die Kolbenanordnung (258) ausgeübte Vorspannkraft zu unterstützen, um den ersten Kolbenkopf (280) und den zweiten Kolbenkopf (282) von den offenen Positionen zurück in die geschlossenen Positionen zu verschieben.

12. Der Verteiler (20) nach einem der Ansprüche 9-11, wobei der Kolbenraum (254) einen ersten Abschnitt (290) mit einem Durchmesser und einen zweiten Abschnitt mit einem Durchmesser, der größer als der Durchmesser des ersten Abschnitts (290) ist, aufweist, wobei der erste Kolbenkopf (280) einen Durchmesser aufweist, und der zweite Kolbenkopf (282) einen Durchmesser aufweist, der größer als der Durchmesser des ersten Kolbenkopfs (280) ist, wobei der erste Kolbenkopf (280) dafür konfiguriert ist, innerhalb des ersten Abschnitts (290) verschoben zu werden, und der zweite Kolbenkopf (282) dafür konfiguriert ist, innerhalb des zweiten Abschnitts (292) verschoben zu werden.

13. Der Verteiler (20) nach einem der Ansprüche 9-12, ferner umfassend: ein fluidbetätigtes Ventil (274), das innerhalb des Absaugströmungswegs (46) angeordnet ist; und einen bedingt fluidisch zwischen dem zentralen Kolbenraumabschnitt (286) und dem fluidbetätigten Ventil (274) kommunizierenden Absaugabschaltkanal (270),
wobei der zweite Kolbenkopf (282) in geschlossener Position dafür konfiguriert ist, die Fluidkommunikation zwischen dem zentralen Kolbenraumabschnitt (286) und dem fluidbetätigten Ventil (274) über den Absaugabschaltkanal (270) zu verhindern und die Fluidkommunikation zwischen dem hinteren Kolbenraumabschnitt (288) und dem fluidbetätigten Ventil (274) über den Absaugabschaltkanal (270) zu ermöglichen, und in offener Position dafür konfiguriert ist, die Fluidkommunikation zwischen dem zentralen Kolbenraumabschnitt (286) und dem fluidbetätigten Ventil (274) über den Absaugabschaltkanal (270) zu ermöglichen und die Fluidkommunikation zwischen dem hinteren Kolbenraumabschnitt (288) und dem fluidbetätigten Ventil (274) über den Absaugabschaltkanal (270) zu verhindern.

14. Der Verteiler (20) nach einem der Ansprüche 9-13, ferner umfassend: einen Druckausgleichskanal (272), der bedingt fluidisch zwischen dem Absaugströmungsweg (46) und dem zentralen Kolbenraumabschnitt (286) kommuniziert, wobei der erste Kolbenkopf (280) in geschlossener Position dafür konfiguriert ist, die Fluidkommunikation zwischen dem Absaugströmungsweg (46) und dem zentralen Kolbenraumabschnitt (286) zu ermöglichen, und in offener Position dafür konfiguriert ist, die Fluidkommunikation zwischen dem Absaugströmungsweg (46) und dem zentralen Kolbenraumabschnitt (286) zu verhindern.

15. Der Verteiler (20) nach einem der Ansprüche 9-14, ferner umfassend: einen Resonator (276), der im Auslasskanal (264) angeordnet ist, wobei der Resonator, wenn die passive Druckoszillationsanordnung (244) im Oszillationsmodus ist, dafür konfiguriert ist, die Fluidkommunikation zwischen dem Kolbenraum (254) und dem Absaugströmungsweg (46) mit einer zweiten Frequenz zu pulsen, die von der ersten Frequenz verschieden ist.

## Revendications

1. Un collecteur (20) comprenant :
une sortie d'aspiration (38) configurée pour être couplée fluidiquement à une source d'aspiration (14) ;
une entrée d'aspiration (36) configurée pour être couplée fluidiquement à un cathéter d'aspiration (12), de sorte qu'un trajet de flux d'aspiration (46) se forme entre le cathéter d'aspiration (12) et la source d'aspiration (14), dans lequel le trajet de flux d'aspiration (46) a une pression absolue de flux libre qui crée une différence de pression négative de flux libre entre la sortie d'aspiration (38) et l'entrée d'aspiration (36) ;
une entrée de décharge (40) configurée pour être couplée fluidiquement à une source de fluide sous pression (16) ; et
un ensemble d'oscillation de pression (244) couplé fluidiquement entre l'entrée de décharge (40) et le trajet de flux d'aspiration (46),
**caractérisé en ce que**
l'ensemble d'oscillation de pression (244) est configuré pour être opéré entre un mode normal qui empêche la communication fluidique entre la source de fluide sous pression (16) et le trajet de flux d'aspiration (46), et un mode oscillatoire qui pulse la communication fluidique entre la source de fluide sous pression (16) et le trajet de flux d'aspiration (46), de sorte qu'une différence de pression négative entre la sortie d'aspiration (38) et l'entrée d'aspiration (36) oscille entre une première différence de pression négative inférieure à la différence de pression de flux libre et une deuxième différence de pression négative supérieure à la différence de pression de flux libre lorsque l'ensemble d'oscillation de pression (244) est opéré en mode oscillatoire.

2. Le collecteur (20) de la revendication 1, dans lequel la première différence de pression négative est une différence de pression d'activation qui déclenche l'ensemble d'oscillation de pression (244) pour augmenter progressivement la différence de pression négative entre la sortie d'aspiration (38) et l'entrée d'aspiration (36), et la deuxième différence de pression négative est une différence de pression de cessation qui déclenche l'ensemble d'oscillation de pression (244) pour diminuer progressivement la différence de pression négative entre la sortie d'aspiration (38) et l'entrée d'aspiration (36).

3. Le collecteur (20) de l'une des revendications 1 et 2, dans lequel la deuxième différence de pression est de 40kPa-90kPa supérieure à la première différence de pression.

4. Le collecteur (20) de l'une des revendications 1 à 3, dans lequel l'ensemble d'oscillation de pression (244) est configuré pour pulser la communication fluidique entre la source de fluide sous pression (16) et le trajet de flux d'aspiration (46) simultanément à une première fréquence et à une deuxième fréquence différente de la première fréquence.

5. Le collecteur (20) de la revendication 4, dans lequel la première fréquence est dans la plage de 0,2Hz à 10Hz, et la deuxième fréquence est dans la plage de 100Hz à 400Hz.

6. Le collecteur (20) selon l'une des revendications 1 à 5, dans lequel l'ensemble d'oscillation de pression (244) est un ensemble d'oscillation de pression passif configuré pour être déclenché afin de basculer automatiquement du mode normal au mode oscillatoire en réponse à un thrombus obstrué (2) dans le cathéter d'aspiration (12).

7. Le collecteur (20) selon la revendication 6, dans lequel l'ensemble d'oscillation de pression passif (244) est configuré pour être déclenché afin de basculer automatiquement du mode oscillatoire au mode normal en réponse à la suppression du thrombus obstrué (2) du cathéter d'aspiration (12).

8. Le collecteur (20) selon l'une des revendications 6 ou 7, dans lequel l'ensemble d'oscillation de pression passif (244) comprend :
une cavité de piston (254) en communication fluidique entre l'entrée de décharge (40) et le trajet de flux d'aspiration (46) ; et
un ensemble de piston (258) disposé de manière coulissante à l'intérieur de la cavité de piston (254), l'ensemble de piston (258) étant configuré pour, lorsque l'ensemble d'oscillation de pression passif (244) est dans le mode normal, empêcher la communication fluidique à travers la cavité de piston (254) entre la source de fluide sous pression (16) et le trajet de flux d'aspiration (46), et lorsque l'ensemble d'oscillation de pression passif (244) est dans le mode oscillatoire, pulser la communication fluidique entre la source de fluide sous pression (16) et le trajet de flux d'aspiration (46) à une première fréquence.

9. Le collecteur (20) selon la revendication 8, dans lequel l'ensemble de piston (258) comprend une tige (278), une première tête de piston (280) fixée à la tige (278), et une deuxième tête de piston (282) fixée à la tige (278) dans une relation espacée avec la première tête de piston (280), formant ainsi une région de cavité de piston avant (284), une région de cavité de piston centrale (286) entre la première tête de piston (280) et la deuxième tête de piston (282), et une région de cavité de piston arrière (288) à l'intérieur de la cavité de piston (254), l'ensemble d'oscillation de pression passif (244) comprenant en outre :
un canal d'entrée (262) en communication fluidique entre l'entrée de décharge (40) et la région de cavité de piston avant (284) ;
un canal de sortie (264) conditionnellement en communication fluidique entre la région de cavité de piston centrale (286) et le trajet de flux d'aspiration (46) ;
un canal de dérivation (266) conditionnellement en communication fluidique entre la région de cavité de piston avant (284) et la région de cavité de piston centrale (286) ;
dans lequel l'ensemble de piston (258) est configuré pour, lorsque l'ensemble d'oscillation de pression passif (244) est dans le mode normal, empêcher la communication fluidique à travers la cavité de piston (254) entre la source de fluide sous pression (16) et le trajet de flux d'aspiration (46) en maintenant la première tête de piston (280) dans une position fermée à l'intérieur de la cavité de piston (254), empêchant ainsi la communication fluidique entre la région de cavité de piston avant (284) et la région de cavité de piston centrale (286) via le canal de dérivation (266), et en maintenant la deuxième tête de piston (282) dans une position fermée à l'intérieur de la cavité de piston (254), empêchant ainsi la communication fluidique entre la région de cavité de piston centrale (286) et le trajet de flux d'aspiration (46) via le canal de sortie (264) ;
dans lequel l'ensemble de piston (258) est configuré pour, lorsque l'ensemble d'oscillation de pression passif (244) est dans le mode oscillatoire, activer la communication fluidique entre la source de fluide sous pression (16) et le trajet de flux d'aspiration (46) à une première fréquence en déplaçant la première tête de piston (280) de la position fermée à une position ouverte à l'intérieur de la cavité de piston (254), permettant ainsi la communication fluidique entre la région de cavité de piston avant (284) et la région de cavité de piston centrale (286) via le canal de dérivation (266), et en déplaçant également la deuxième tête de piston (282) de la position fermée à une position ouverte à l'intérieur de la cavité de piston (254), permettant ainsi la communication fluidique entre la région de cavité de piston centrale (286) et le trajet de flux d'aspiration (46), puis en déplaçant la première tête de piston (280) et la deuxième tête de piston (282) des positions ouvertes vers les positions fermées.

10. Le collecteur (20) selon la revendication 9, dans lequel l'ensemble d'oscillation de pression passif (244) comprend en outre un ressort (260) configuré pour appliquer une force de biaisage à l'ensemble de piston (258) pendant le fonctionnement de l'ensemble d'oscillation de pression passif (244) en mode normal, maintenant la première tête de piston (280) et la deuxième tête de piston (282) dans les positions fermées à l'intérieur de la cavité de piston (254), dans lequel l'ensemble de piston (258), pendant le fonctionnement de l'ensemble d'oscillation de pression passif (244) en mode oscillatoire, est configuré pour, en réponse à la pression appliquée par le fluide provenant de la source de fluide sous pression (16) à l'ensemble de piston (258) via le canal d'entrée (262), surmonter la force de biaisage appliquée par le ressort (260) à l'ensemble de piston (258) pour déplacer la première tête de piston (280) et la deuxième tête de piston (282) des positions fermées aux positions ouvertes à l'intérieur de la cavité de piston (254), puis compléter la force de biaisage appliquée par le ressort (260) à l'ensemble de piston (258) pour déplacer la première tête de piston (280) et la deuxième tête de piston (282) des positions ouvertes vers les positions fermées.

11. Le collecteur (20) selon la revendication 10, dans lequel l'ensemble d'oscillation de pression passif (244) comprend en outre un canal de prise de pression (268) en communication fluidique entre le trajet de flux d'aspiration (46) et la région arrière de la cavité de piston (288), dans lequel l'ensemble de piston (258), pendant le fonctionnement de l'ensemble d'oscillation de pression passif (244) en mode oscillatoire, est configuré pour, en réponse à la pression appliquée par le fluide provenant du trajet de flux d'aspiration (46) à l'ensemble de piston (258) via le canal de prise de pression (268), compléter la force de biaisage appliquée par le ressort (260) à l'ensemble de piston (258) afin de déplacer la première tête de piston (280) et la deuxième tête de piston (282) des positions ouvertes vers les positions fermées.

12. Le collecteur (20) selon l'une des revendications 9 à 11, dans lequel la cavité de piston (254) comprend une première partie (290) ayant un diamètre, et une deuxième partie ayant un diamètre supérieur à celui de la première partie (290), la première tête de piston (280) ayant un diamètre, et la deuxième tête de piston (282) ayant un diamètre supérieur à celui de la première tête de piston (280), dans lequel la première tête de piston (280) est configurée pour être déplacée à l'intérieur de la première partie (290), et la deuxième tête de piston (282) est configurée pour être déplacée à l'intérieur de la deuxième partie (292).

13. Le collecteur (20) selon l'une des revendications 9 à 12, comprenant en outre : une vanne actionnée par fluide (274) disposée à l'intérieur du trajet de flux d'aspiration (46) ; et un canal d'arrêt d'aspiration (270) conditionnellement en communication fluidique entre la région centrale de la cavité de piston (286) et la vanne actionnée par fluide (274),
dans lequel la deuxième tête de piston (282), lorsqu'elle est dans la position fermée, est configurée pour empêcher la communication fluidique entre la région centrale de la cavité de piston (286) et la vanne actionnée par fluide (274) via le canal d'arrêt d'aspiration (270), et permettre la communication fluidique entre la région arrière de la cavité de piston (288) et la vanne actionnée par fluide (274) via le canal d'arrêt d'aspiration (270), et lorsqu'elle est dans la position ouverte, est configurée pour permettre la communication fluidique entre la région centrale de la cavité de piston (286) et la vanne actionnée par fluide (274) via le canal d'arrêt d'aspiration (270), et empêcher la communication fluidique entre la région arrière de la cavité de piston (288) et la vanne actionnée par fluide (274) via le canal d'arrêt d'aspiration (270).

14. Le collecteur (20) selon l'une des revendications 9 à 13, comprenant en outre un canal d'égalisation de pression (272) conditionnellement en communication fluidique entre le trajet de flux d'aspiration (46) et la région centrale de cavité de piston (286), dans lequel la première tête de piston (280), lorsqu'elle est dans la position fermée, est configurée pour permettre la communication fluidique entre le trajet de flux d'aspiration (46) et la région centrale de cavité de piston (286), et, lorsqu'elle est dans la position ouverte, est configurée pour empêcher la communication fluidique entre le trajet de flux d'aspiration (46) et la région centrale de cavité de piston (286).

15. Le collecteur (20) selon l'une des revendications 9 à 14, comprenant en outre un résonateur (276) disposé dans le canal de sortie (264), dans lequel le résonateur, lorsque l'ensemble d'oscillation de pression passif (244) est en mode oscillatoire, est configuré pour pulser la communication fluidique entre la cavité de piston (254) et le trajet de flux d'aspiration (46) à une deuxième fréquence différente de la première fréquence.
